# EUROPÄISCHE PATENTSCHRIFT

(11) **EP 3 701 912 B1**
(45) Veröffentlichungstag und Bekanntmachung des Hinweises auf die Patenterteilung: **27.01.2021**
(21) Anmeldenummer: 20151621.8
(22) Anmeldetag: 14.01.2020
(51) Int. Cl.: A61F 2/30, A61F 2/36, A61F 2/46

(54) **FEMORALER HÜFTGELENKSPACER MIT SPÜLVORRICHTUNG**
FEMORAL HIP JOINT SPACER WITH FLUSHING DEVICE
ESPACEUR FÉMORALE DE L'ARTICULATION DE LA HANCHE POURVU DE DISPOSITIF DE RINÇAGE

(30) Priorität: 16.01.2019 DE 102019101081
(43) Veröffentlichungstag der Anmeldung: 02.09.2020
(73) Patentinhaber: Heraeus Medical GmbH, 61273 Wehrheim (DE)
(72) Erfinder: Vogt, Sebastian, 61273 Wehrheim (DE); Kluge, Thomas, 61273 Wehrheim (DE)
(74) Vertreter: Schultheiss & Sterzel Patentanwälte PartG mbB

(56) Entgegenhaltungen:
- EP-A1- 3 542 759
- WO-A1-2017/178951
- US-A1- 2011 015 754
- US-A1- 2013 211 369
- US-A1- 2018 028 320

## Beschreibung

Die Erfindung betrifft einen femoralen Hüftgelenkspacer mit Spülvorrichtung, wie in den Ansprüchen beschrieben, zum zeitweisen Ersetzen zumindest eines Teils eines Hüftgelenks, der für die Interimsphase von zweizeitigen septischen Revisionen von Hüftgelenk-Endoprothesen bestimmt ist. Der Hüftgelenkspacer kann besonders bei solchen zweizeitigen septischen Revisionen Anwendung finden, bei denen zwei oder mehrere mikrobielle Keime ursächlich für eine Infektion der Hüftgelenk-Endoprothese und des umgebenden Gewebes sind.

Hüftgelenk-Endoprothesen werden in großem Umfang weltweit implantiert. Leider kommt es in einem geringen Prozentsatz vor, dass Hüftgelenk-Endoprothesen von mikrobiellen Keimen, besonders Gram-positiven Bakterien als auch Gram-negativen Bakterien und in sehr geringem Umfang von Hefen und Pilzen, besiedelt werden. Diese mikrobiellen Keime, hauptsächlich typische Hautkeime wie Staphylococcus aureus und Staphylococcus epidermidis, können während einer chirurgischen Operation (OP) in den Patienten gelangen. Daneben ist es auch möglich, dass mikrobielle Keime hämatogen Hüftgelenk-Endoprothesen erreichen. Bei einer Besiedlung von Hüftgelenk-Endoprothesen mit mikrobiellen Keimen wird das umliegende Knochen- und Weichgewebe mit infiziert und von den mikrobiellen Keimen geschädigt.

Stand der Technik sind vor allem zwei Behandlungsverfahren für infizierte Hüftgelenk-Endoprothesen, die einzeitige septische Revision und die zweizeitige septische Revision. Daneben gibt es noch eine Anzahl weiterer Behandlungsverfahren, wie zum Beispiel die Anwendung von Saug-Spül-Drainagen.

Bei der einzeitigen Revision wird innerhalb einer OP zuerst die infizierte Hüftgelenk-Endoprothesen entfernt, anschließend radikal debridiert und dann wird eine Revisions-Hüftgelenk-Endoprothese implantiert.

Bei zweizeitigen septischen Revisionen wird in einer ersten OP zuerst die infizierte Hüftgelenk-Endoprothesen entfernt, dann wird debridiert und anschließend erfolgt die Implantation eines Hüftgelenkspacers. Der Hüftgelenkspacer besteht aus einem Schaft, einem Kragen, einem Hals und einem Kugelkopf und ist den Hüftgelenk-Endoprothesen in Form und Größe nachgebildet. Der Hüftgelenkspacer wird mit Knochenzement am proximalen Femur beziehungsweise im Femurkanal verankert. Der Hüftgelenkspacer verbleibt bis zu mehreren Wochen im Patienten bis die Entzündung abgeklungen ist und die klinischen Entzündungsmarker zurückgegangen sind. Dann wird in einer zweiten OP der Hüftgelenkspacer entfernt und nach erneutem Debridement eine Revisions-Hüftgelenk-Endoprothese implantiert.

Die US 2010/0042213 A1 offenbart eine Hüftgelenkprothese mit einem Reservoir einer Flüssigkeit im Inneren des Implantats. Aus der WO 2017/178951 A1 ist ein Hüftspacer mit Vertiefungen bekannt, wobei in den Vertiefungen eine Substanz zur Behandlung des Knochens eingebracht werden kann. In dem Patent US 6 245 111 B1 wird eine Hüftgelenksprothese vorgeschlagen, deren Oberflächen mit einem Antibiotikum beschichtet sind. Das Patent US 5 681 289 offenbart eine Einrichtung zum Verteilen eines flüssigen Wirkstoffs mit Hilfe einer Blase im Inneren der Einrichtung. Keine der genannten Prothesen ist zum Erzeugen eines Spülkreislaufs geeignet.

Es ist bekannt, mit Antibiotika ausgerüstete Spacer zu verwenden. Hüftgelenkspacer können einerseits vom OP-Personal während der OP selbst aus PMMA-Knochenzementpulver, Antibiotika und Monomerflüssigkeit hergestellt werden, beispielsweise mit einer Spacerform, wie sie beispielsweise in den Patenten DE 10 2015 104 704 B4 oder EP 2 617 393 B1 beschrieben sind, und andererseits ist es auch üblich, industriell aus Knochenzement vorgefertigte Hüftgelenkspacer einzusetzen.

Bei den bisherigen Spacern werden dem Zementpulver vor der eigentlichen Spacerherstellung Antibiotika zugesetzt. Mit diesem antibiotisch modifizierten Knochenzementpulver werden anschließend Spacer gegossen, die dann durch Polymerisation mit Hilfe einer dem Zementpulver zugesetzten Monomerflüssigkeit aushärten. Der Knochenzementteig schließt dabei die Antibiotika im Wesentlichen ein. Nur die in den oberflächennahen Bereichen befindlichen Antibiotika-Partikel werden bei Einwirkung von Körperflüssigkeiten, wie Wundsekret, herausgelöst. Die Wirkstofffreisetzung ist initial am höchsten und nimmt dann im Verlauf von mehreren Tagen ab. Danach werden nur noch geringe Mengen der Antibiotika freigesetzt. Die Hauptmenge der zugesetzten Antibiotika verbleibt im ausgehärteten Knochenzement der Spacer. Eine nachträgliche Veränderung der Art und der Anzahl der eingesetzten Antibiotika ist nach der Spacerherstellung bzw. nach der Implantation bei den bisherigen aus Knochenzement gefertigten Spacern nicht möglich. Weiterhin ist die Einstellung einer definierten Konzentration an antimikrobiellen Wirkstoffen im Wundsekret beziehungsweise der den Spacer umgebenden Körperflüssigkeit ebenfalls nicht möglich.

Im Patent EP 1 991 170 B1 wird ein Hüftgelenkspacer beschrieben, bei dem der Kugelkopf und der Schaft vom Anwender zusammengesteckt werden können, wobei der Kugelkopf einen ersten Wirkstoff enthält und der Schaft mit einem zweiten Wirkstoff ausgerüstet ist.

Ein weiterer Hüftgelenkspacer wird in der US 2011/0015754 A1 offenbart. Bei diesem Spacersystem sind in dem Kugelkopf zwei zylinderförmige Hohlräume vorgesehen, die mit einer Schmalseite auf die Kugeloberfläche münden. Die beiden Hohlräume sind jeweils mit einem flüssigkeitsdurchlässigen Deckel verschlossen. Beide Hohlräume können mit antibiotischen Lösungen befüllt werden. Nach der Implantation migrieren die antibiotischen Lösungen durch die flüssigkeitsdurchlässigen Deckel auf die Oberfläche des Spacers. Die nicht vorveröffentlichte US 2019/0290833 A1 offenbart einen spülbaren Hüftgelenkspacer, mit dem ein Flüssigkeitskreislauf erzeugbar ist.

In der WO 2016/205077 A1 und der US 8 900 322 B2 werden Spacer mit einer Spülfunktion beschrieben. Bei den dort beschriebenen Hüftgelenkspacern sind eine Vielzahl von Austrittsöffnungen an der Außenseite eines Spacerschafts angeordnet. Weiterhin befinden sich die Öffnungen in Vertiefungen, sogenannten Valleys, von Finnen. Die Finnen dienen der Verankerung im Femurkanal und der Beabstandung der Öffnungen von den Wänden des Femurkanals, damit die Öffnungen nicht von Knochengewebe abgedeckt werden können. Beim Debridement im Rahmen des zweiseitigen Wechsels wird umfangreich und radikal infiziertes und nekrotisches Gewebe entfernt. Der Durchmesser des Femurkanals kann daher in weiten Grenzen variieren. Eine Verankerung des Hüftgelenkspacers über Finnen ist jedoch nur dann möglich, wenn die radialen Abmessungen der Finnen dem Durchmesser des Femurkanals entsprechen. Weder in der WO 2016/205077 A1 noch in der US 8 900 322 B2 sind Vorrichtungen beschrieben, die eine Anpassung der Verankerung entsprechend den Abmessungen des Femurkanals zulassen. Die nachveröffentlichte US 2019/0290833 A1 offenbart einen femoralen Hüftgelenkspacer mit einer Spülvorrichtung, bei dem durch zwei Leitungen von außen Spülflüssigkeit in den Spacer hinein und aus dem Spacer hinaus geleitet werden, um zumindest einen Spülflüssigkeitskreislauf zu erzeugen.

Bei der zweizeitigen septischen Revision werden bei der Implantation der Hüftgelenkspacer auch Drainagen eingesetzt, die zur Ableitung von Wundsekret, Blut und Debris bestimmt sind. Die Drainagen verbleiben bis zu mehreren Tagen im Patienten. Die von dem Spacer freigesetzten antibiotischen Wirkstoffe werden vom Wundsekret aufgenommen und über die Drainage nach außen abgeführt. Dadurch geht ein Teil der antimikrobiellen Wirkstoffe zum Schutz der Spaceroberfläche vor mikrobieller Besiedlung verloren.

Es wurde im Rahmen der vorliegenden Erfindung erkannt, dass es wünschenswert wäre, wenn die Spaceroberfläche von einer antimikrobiellen Wirkstofflösung umgeben wäre, deren Wirkstoffkonzentration exakt eingestellt werden kann und deren Konzentration über mehrere Tage unabhängig vom Wundsekretstrom erhalten bleiben würde. Weiterhin wäre es wünschenswert, dass man die Art und die Anzahl der mikrobiellen Wirkstoffe auch nach der Implantation des Hüftgelenkspacers variieren kann, um zum Beispiel auf erst später nachgewiesene mikrobielle Keime reagieren zu können. Gleichzeitig soll der Patient das Hüftgelenk bewegen können, um eine Verkürzung der Sehnen und der Muskulatur und eine Degeneration der Muskulatur zu verhindern und so die Rehabilitation zu verkürzen.

Die Aufgabe der vorliegenden Erfindung besteht also darin, die Nachteile des Stands der Technik zu überwinden. Insbesondere besteht die Aufgabe der Erfindung in der Entwicklung eines temporären femoralen Hüftgelenkspacers, mit dem eine medizinische Spülflüssigkeit gezielt im Bereich der Hüfte eingesetzt werden kann und bei dem die medizinische Spülflüssigkeit möglichst die ganze Oberfläche des Prothesenkörpers des Hüftgelenkspacers erreichen kann, um eine medizinische Behandlung des gesamten an die Oberfläche des Prothesenkörpers grenzenden Gewebes bis auf die unmittelbare Verankerung am Femur zu ermöglichen. Gleichzeitig soll der Hüftgelenkspacer eine Beweglichkeit des Hüftgelenks im beim Patienten eingesetzten Zustand ermöglichen.

Die Aufgabe der Erfindung ist es dabei, einen artikulierenden Hüftgelenkspacer zu entwickeln, der für die Interimsphase von zweizeitigen septischen Revisionen von Hüftgelenk-Endoprothesen bestimmt ist und der hierfür nutzbringende Eigenschaften aufweist. Der Hüftgelenkspacer soll den Raum nach der Entfernung der Hüftgelenk-Endoprothese und dem nachfolgenden Debridement so ausfüllen, dass eine Rückbildung des Bandapparates und der Muskulatur verhindert wird. Der zu entwickelnde Hüftgelenkspacer soll es ermöglichen, dass die artikulierende Spaceroberfläche, das den Hüftgelenkspacer umgebende Weichgewebe und zumindest ein Teil des umgebenden Knochengewebes mit antiseptischen oder antibiotischen Spülflüssigkeiten kontinuierlich oder auch diskontinuierlich gespült werden können. Der Hüftgelenkspacer soll mit dem Knochengewebe des proximalen Femurs in der Weise mit Knochenzement verbunden werden können, dass der Austritt der Spülflüssigkeit aus dem Schaft oder im Bereich des Schafts des Hüftgelenkspacers und auch die Aufnahme der Spülflüssigkeit in den Hüftgelenkspacer zur Ableitung nicht gestört oder unterbrochen wird. Der Hüftgelenkspacer soll ferner möglichst so beschaffen sein, dass nach einer Beendigung der Spülungen mit der Spülflüssigkeit die Spülflüssigkeitszuführung und die Spülflüssigkeitsableitung entfernt werden können, ohne dass die Artikulation des Hüftgelenkspacers beeinträchtigt wird.

Zur Vermeidung von Kreislaufproblemen und Embolien ist es medizinisch üblich, Patienten in der Interimsphase zu mobilisieren. Daher ist es erforderlich, dass der zu entwickelnden Hüftgelenkspacer mechanisch im proximalen Femur fixiert werden kann. Die mechanische Befestigung ist zwingend notwendig, um unerwünschte mechanische Beanspruchen, insbesondere Biegebeanspruchungen des proximalen Femurs zu vermeiden. Biegebeanspruchungen des Femurs durch nicht fixierte Hüftgelenkspacer können ansonsten zu Femurbrüchen führen.

Die Aufgaben der Erfindung werden gelöst durch einen femoralen Hüftgelenkspacer zum zeitweisen Ersetzen eines Teils eines Hüftgelenks, der Hüftgelenkspacer aufweisend einen Prothesenkörper, der Prothesenkörper aufweisend einen Kugelkopf mit einer Gleitfläche, einen Hals, der an seiner proximalen Seite mit dem Kugelkopf verbunden ist, einen Schaft, der auf einer dem Kugelkopf gegenüberliegenden distalen Seite des Halses mit dem Hals verbunden ist, und eine Verankerungshülse, die den Schaft auf einer proximalen Seite des Schafts mit einer umlaufenden Befestigungsfläche umschließt und die mit dem Schaft verbunden ist,
der Hüftgelenkspacer ferner aufweisend eine Spülflüssigkeits-Einlassöffnung in einer Oberfläche des Prothesenkörpers,
eine Spülflüssigkeits-Auslassöffnung in der Oberfläche des Prothesenkörpers,
zumindest eine Spülflüssigkeitsaustrittsöffnung auf einer distalen Seite des Schafts und zumindest eine Spülflüssigkeitseintrittsöffnung am Kugelkopf oder am Hals, wobei
die zumindest eine Spülflüssigkeitsaustrittsöffnung im Inneren des Prothesenkörpers mit der Spülflüssigkeits-Einlassöffnung flüssigkeitsdurchlässig verbunden ist und nicht flüssigkeitsdurchlässig mit der Spülflüssigkeits-Auslassöffnung verbunden ist und
die zumindest eine Spülflüssigkeitseintrittsöffnung im Inneren des Prothesenkörpers mit der Spülflüssigkeits-Auslassöffnung flüssigkeitsdurchlässig verbunden ist und nicht flüssigkeitsdurchlässig mit der Spülflüssigkeits-Einlassöffnung verbunden ist und wobei
im Inneren der Verankerungshülse ein zweiseitig offener Hohlraum ausgebildet ist, der eine proximale Seite der Verankerungshülse mit einer distalen Seite der Verankerungshülse flüssigkeitsdurchlässig verbindet.

In der vorliegenden Patentanmeldung werden die Richtungsbezeichnungen "proximal", "distal" und "lateral" sowie die Ebenen-bezeichnungen "Sagittalebene", "Frontalebene" und "Transversalebene" in Bezug auf den Hüftgelenkspacer so verwendet, wie dies bei dem im Patienten eingesetzten Zustand als anatomische Hauptrichtung oder als Körperebene zu verstehen wäre. Dabei bedeutet "proximal" zum Körperzentrum hin und "distal" vom Körperzentrum entfernt.

Die Befestigungsfläche ist zur Verbindung mit einem Femur vorgesehen und ist vorzugsweise zu diesem Zweck in ein proximales Ende des präparierten Femurs beziehungsweise in den Femurkanal einführbar.

Bevorzugt weist die Verankerungshülse eine geschlossene Mantelfläche auf. Ebenfalls bevorzugt umschließt die Befestigungsfläche den Schaft vollständig.

Es kann vorgesehen sein, dass der Hüftgelenkspacer aus Metall, Kunststoff, Elastomeren, Keramik oder Kombinationen dieser Werkstoffe gefertigt ist.

Bevorzugt kann vorgesehen sein, dass der Hüftgelenkspacer zur Anwendung zumindest eines antibiotischen und/oder antimykotischen Wirkstoffs geeignet ist, der eine Polymerisation oder eine radikalische Polymerisation von PMMA verhindert oder beeinträchtigt. Insbesondere kann vorgesehen sein, dass der Hüftgelenkspacer zur Anwendung von Rifampicin und Metronidazol geeignet ist.

Der Prothesenkörper ist vorzugsweise einteilig. Besonders bevorzugt ist der Prothesenkörper einteilig aus einem biokompatiblen Material, wie Polymethylmethacrylat (PMMA), gefertigt, wobei ganz besonders bevorzugt das PMMA wenigsten ein aus dem PMMA lösbares Antibiotikum und/oder Antimykotikum enthält.

Alternativ kann aber auch vorgesehen sein, dass der Hüftgelenkspacer zweiteilig ausgebildet ist, und einen Kopfteil umfassend den Kugelkopf und einen von dem Kopfteil lösbaren Schaft aufweist, wobei die Verankerungshülse mit dem Schaft unlösbar verbunden ist.

Die zumindest eine Spülflüssigkeitsaustrittsöffnung und die zumindest eine Spülflüssigkeitseintrittsöffnung sind vorzugsweise in der Oberfläche des Prothesenkörpers angeordnet.

Es kann vorgesehen sein, dass der Hüftgelenkspacer ein erstes röhrenförmiges und flüssigkeitsdurchlässiges Verbindungsmittel zum Abführen der Spülflüssigkeit aus dem Prothesenkörper aufweist, wobei das erste Verbindungsmittel mit der Spülflüssigkeits-Auslassöffnung flüssigkeitsdurchlässig verbunden oder verbindbar ist, und der Hüftgelenkspacer ein zweites röhrenförmiges und flüssigkeitsdurchlässiges Verbindungsmittel zum Zuführen einer medizinischen Spülflüssigkeit in den Prothesenkörper aufweist, wobei das zweite Verbindungsmittel mit der Spülflüssigkeits-Einlassöffnung flüssigkeitsdurchlässig verbunden oder verbindbar ist.

Hierdurch kann die Spülflüssigkeit auf einfache Weise in den Prothesenkörper eingespeist werden und aus dem Prothesenkörper abgeführt werden.

Die Spülflüssigkeit kann theoretisch auch zunächst durch das zweite Verbindungsmittel eingeleitet werden und durch das erste Verbindungsmittel abgeführt werden und anschließend durch das erste Verbindungsmittel eingeleitet werden und durch das zweite Verbindungsmittel abgeführt werden. Der Hüftgelenkspacer wird dann alternierend betrieben. Es wird jedoch offenbarungsgemäß bevorzugt, dass der Hüftgelenkspacer nur in einer Fließrichtung der Spülflüssigkeit betrieben wird beziehungsweise zu betreiben ist.

Vorzugsweise ist das erste röhrenförmige flüssigkeitsdurchlässige Verbindungsmittel ein Schlauch mit einem Adapter oder einem anderen Anschluss.

Vorzugsweise ist das zweite röhrenförmige flüssigkeitsdurchlässige Verbindungsmittel ein Schlauch mit einem Adapter oder einem anderen Anschluss.

Es kann bei offenbarungsgemäßen Hüftgelenkspacern mit dem ersten und dem zweiten Verbindungsmittel vorgesehen sein, dass das erste Verbindungsmittel an der zur Verbindung mit der Spülflüssigkeits-Einlassöffnung abgewandten Seite und das zweite Verbindungsmittel an der zur Verbindung mit der Spülflüssigkeits-Auslassöffnung abgewandten Seite jeweils einen Adapter aufweist, insbesondere jeweils einen Luer-Lock-Adapter aufweist.

Es kann bei erfindungsgemäßen Hüftgelenkspacern vorgesehen sein, dass in dem ersten Verbindungsmittel oder in der Spülflüssigkeits-Auslassöffnung ein erstes Ventilelement angeordnet ist, das ein Rückfließen der Spülflüssigkeit in das erste Verbindungsmittel verhindert, und/oder in dem zweiten Verbindungsmittel oder in der Spülflüssigkeits-Einlassöffnung ein zweites Ventilelement angeordnet ist, das ein Rückfließen der Spülflüssigkeit in das zweite Verbindungsmittel verhindert. Dabei kann bevorzugt vorgesehen sein, dass das erste und/oder das zweite Ventilelement ausgewählt sind aus einem Rückschlagventil, einem Kugelventil mit Feder, einem Lippenventil, einem Bunsenventil oder einem Plattenventil.

Dadurch kann ein umlaufender Kreislauf der medizinischen Spülflüssigkeit vorgegeben werden. Zudem kann so ein Rücklaufen der benutzten medizinischen Spülflüssigkeit verhindert werden.

Gemäß einer bevorzugten Weiterbildung der vorliegenden Erfindung kann vorgesehen sein, dass die zumindest eine Spülflüssigkeitsaustrittsöffnung und die zumindest eine Spülflüssigkeitseintrittsöffnung in der Oberfläche des Prothesenkörpers außerhalb der umlaufenden Befestigungsfläche angeordnet sind.

Hiermit wird sichergestellt, dass die aus der zumindest einen Spülflüssigkeitsaustrittsöffnung austretende Spülflüssigkeit beziehungsweise die durch die zumindest eine Spülflüssigkeitseintrittsöffnung einströmende Spülflüssigkeit nicht die Verbindung zum Femur beeinträchtigen kann und dass andererseits der Zement, der zur Befestigung des Hüftgelenkspacers am Femur beziehungsweise im Femurkanal verwendet wird, nicht eine oder mehrere der zumindest einen Spülflüssigkeitsaustrittsöffnung und/oder der zumindest einen Spülflüssigkeitseintrittsöffnung ungewollt verschließt.

Ferner kann vorgesehen sein, dass die Spülflüssigkeits-Einlassöffnung und/oder die Spülflüssigkeits-Auslassöffnung am Kugelkopf oder am Hals des Prothesenkörpers angeordnet sind, wobei vorzugsweise die Spülflüssigkeits-Einlassöffnung und/oder die Spülflüssigkeits-Auslassöffnung an einer lateralen Seite des Halses des Prothesenkörpers angeordnet sind.

An diesen Stellen des Prothesenkörpers lassen sich die Verbindungsschläuche besonders einfach mit dem Prothesenkörper verbinden, ohne dass dies die Funktion des Prothesenkörpers beeinträchtigt und ohne dass dies die Bewegung des Gelenks erschwert.

Des Weiteren kann vorgesehen sein, dass der Schaft hohlzylinderförmig ist, wobei eine Leitung im Inneren des Schafts ausgebildet ist, die die zumindest eine Spülflüssigkeitsaustrittsöffnung mit der Spülflüssigkeits-Einlassöffnung flüssigkeitsdurchlässig verbindet.

Hierdurch kann die Spülflüssigkeit innerhalb des Schafts von der Spülflüssigkeits-Einlassöffnung zu der zumindest einen Spülflüssigkeitsaustrittsöffnung auf der distalen Seite des Schafts geleitet werden.

Bevorzugt kann auch vorgesehen sein, dass die Befestigungsfläche begrenzt ist, wobei die Befestigungsfläche zur Aufnahme von Knochenzementteig geeignet ist.

Alternativ kann auch vorgesehen sein, dass die Befestigungsfläche mit zwei umlaufenden, sich aus der Oberfläche des Prothesenkörpers erhebenden Stegen an einer proximalen Seite und an einer distalen Seite der Verankerungshülse begrenzt ist, wobei die Befestigungsfläche zur Aufnahme von Knochenzementteig innerhalb der Stege geeignet ist.

Gemäß einer weiteren Alternative kann auch vorgesehen sein, dass die Befestigungsfläche mit einem umlaufenden, sich aus der Oberfläche des Prothesenkörpers erhebenden Steg an einer distalen Seite der Verankerungshülse und einem umlaufenden, sich aus der Oberfläche des Prothesenkörpers erhebenden Kragen an einer proximalen Seite der Verankerungshülse begrenzt ist, wobei die Befestigungsfläche zur Aufnahme von Knochenzementteig innerhalb des Stegs und des Kragens geeignet ist.

Durch diese drei alternativen Maßnahmen kann ein begrenzter und dadurch ein bestimmter Bereich zur Befestigung des Prothesenkörpers im Femurkanal verwendet werden. Bei korrekter Anwendung des Hüftgelenkspacers kann dadurch verhindert werden, dass die Spülflüssigkeits-Einlassöffnung und die Spülflüssigkeits-Auslassöffnung sowie die zumindest eine Spülflüssigkeitsaustrittsöffnung und die zumindest eine Spülflüssigkeitseintrittsöffnung mit Knochenzement bedeckt werden und dadurch in ihrer Funktion beeinträchtigt werden. Insbesondere kann verhindert werden, dass der ausgehärtete Knochenzement, mit dem der Hüftgelenkspacer im Femur verankert ist, ein Abziehen beziehungsweise ein Lösen des ersten und des zweiten Verbindungsmittels vom Prothesenkörper verhindert.

Zur Optimierung der vollständigen Behandlung kann vorgesehen sein, dass eine erste Spülflüssigkeitsaustrittsöffnung der wenigstens einen Spülflüssigkeitsaustrittsöffnung am distalen Ende des Schafts angeordnet ist.

Hierdurch wird erreicht, dass die Spülflüssigkeit auch am distalen Ende des Schafts mit der medizinischen Spülflüssigkeit spülen kann, so dass auch ein Durchspülen des Femurs bis in die erreichbare Tiefe erfolgen kann.

Dabei kann vorgesehen sein, dass eine zweite Spülflüssigkeitsaustrittsöffnung auf der proximalen Seite des Kugelkopfs angeordnet ist, wobei die eine erste Spülflüssigkeitsaustrittsöffnung und die zweite Spülflüssigkeitsaustrittsöffnung im Inneren des Prothesenkörpers mit der Spülflüssigkeits-Einlassöffnung flüssigkeitsdurchlässig verbunden sind.

Mit der zweiten Spülflüssigkeitsaustrittsöffnung wird erreicht, dass der Kugelkopf auch an seiner Gleitfläche stärker mit der medizinischen Spülflüssigkeit umspült wird.

Es kann auch vorgesehen sein, dass die zumindest eine Spülflüssigkeitseintrittsöffnung am Hals des Prothesenkörpers und/oder an der distalen Seite des Kugelkopfs angeordnet ist.

Hierdurch wird der Strom der Spülflüssigkeit auf einen Bereich gelenkt, der von außen zugänglich ist, und der für eine Behandlung wichtig ist. Die Spülflüssigkeit kann dabei durch den Hohlraum in der Verankerungshülse fließen und so den gesamten Prothesenkörper umfließen. Zudem kann durch diese Maßnahme die zumindest eine Spülflüssigkeitseintrittsöffnung dicht oder sogar am gleichen Ort wie die Spülflüssigkeits-Auslassöffnung angeordnet werden, so dass keine oder nur eine sehr kurze Leitung zur Verbindung der zumindest einen Spülflüssigkeitseintrittsöffnung mit der Spülflüssigkeits-Auslassöffnung notwendig ist.

Bevorzugt kann die zumindest eine Spülflüssigkeitseintrittsöffnung und die Spülflüssigkeits-Auslassöffnung in einem gemeinsamen Sackloch in der Oberfläche des Prothesenkörpers ausgebildet sein.

Des Weiteren kann vorgesehen sein, dass die Verankerungshülse zwischen der zumindest einen Spülflüssigkeitsaustrittsöffnung und der zumindest einen Spülflüssigkeitseintrittsöffnung angeordnet ist, so dass die Spülflüssigkeit bei einer flüssigkeitsdichten Verbindung der umlaufenden Befestigungsfläche mit einem umgebenden Femurkanal nur durch den zweiseitig offenen Hohlraum von der zumindest einen Spülflüssigkeitsaustrittsöffnung zu der zumindest einen Spülflüssigkeitseintrittsöffnung fließen kann.

Hierdurch wird sichergestellt, dass die Spülflüssigkeit auch bei einer vollflächigen Verbindung der umlaufenden Befestigungsfläche zum Femurkanal alle Oberflächen des Prothesenkörpers erreichen kann.

Ferner kann auch vorgesehen sein, dass an der Spülflüssigkeits-Einlassöffnung im Inneren des Prothesenkörpers oder an der Oberfläche des Prothesenkörpers eine selbstdichtende Kupplung angeordnet ist und an der Spülflüssigkeits-Auslassöffnung im Inneren des Prothesenkörpers oder an der Oberfläche des Prothesenkörpers eine selbstdichtende Kupplung angeordnet ist, wobei vorzugsweise Verbindungsmittel lösbar mit der Spülflüssigkeits-Einlassöffnung und mit der Spülflüssigkeits-Auslassöffnung verbunden oder verbindbar sind.

Hierdurch verschließen sich die Spülflüssigkeits-Einlassöffnung und die Spülflüssigkeits-Auslassöffnung beziehungsweise die Flüssigkeitsleitungen dahinter selbsttätig, wenn ein Verbindungsmittel, wie insbesondere das erste Verbindungsmittel oder das zweite Verbindungsmittel, vom Prothesenkörper abgezogen beziehungsweise getrennt wird. Dadurch kann eine (oder auch mehrere) im Prothesenkörper vorhandene fluidleitende Verbindung verschlossen werden, wenn keine Spülflüssigkeit mehr durch den Hüftgelenkspacer geleitet werden soll.

Gemäß einer Weiterbildung der vorliegenden Erfindung kann vorgesehen sein, dass die Summe der Querschnittsflächen aller der zumindest einen Spülflüssigkeitseintrittsöffnung zusammen zumindest genauso groß ist wie die Querschnittsfläche der Spülflüssigkeits-Einlassöffnung und/oder die Summe der Querschnittsflächen aller der zumindest einen Spülflüssigkeitsaustrittsöffnung zumindest genauso groß ist wie die Querschnittsfläche der Spülflüssigkeits-Auslassöffnung.

Hierdurch kann ein Staudruck im Inneren des Prothesenkörpers vermieden werden.

Bevorzugt kann ferner vorgesehen sein, dass in einer ersten Leitung innerhalb des Prothesenkörpers, die die zumindest eine Spülflüssigkeitseintrittsöffnung mit der Spülflüssigkeits-Auslassöffnung flüssigkeitsdurchlässig verbindet, ein erstes Ventil angeordnet ist, das nur durch Anlegen eines Unterdrucks an der Spülflüssigkeits-Auslassöffnung zu öffnen ist und das ein Zurückfließen der Spülflüssigkeit in die erste Leitung verhindert.

Auch kann vorgesehen sein, dass in einer zweiten Leitung innerhalb des Prothesenkörpers, die die zumindest eine Spülflüssigkeitsaustrittsöffnung mit der Spülflüssigkeits-Einlassöffnung flüssigkeitsdurchlässig verbindet, ein zweites Ventil angeordnet ist, das nur durch Anlegen eines Unterdrucks an der Spülflüssigkeits-Einlassöffnung zu öffnen ist und das ein Zurückfließen der Spülflüssigkeit in die zweite Leitung verhindert.

Auch durch diese beiden Maßnahmen kann ein Rücklaufen der medizinischen Spülflüssigkeit verhindert werden. Zudem kann so sichergestellt werden, dass ohne die Verbindungsmittel noch ein Austausch enthaltener Spülflüssigkeit mit umgebenden Flüssigkeiten stattfindet.

Es kann auch vorgesehen sein, dass in dem Prothesenkörper die Spülflüssigkeits-Einlassöffnung, die Spülflüssigkeits-Auslassöffnung, die zumindest eine Spülflüssigkeitsaustrittsöffnung und die zumindest eine Spülflüssigkeitseintrittsöffnung sowie die flüssigkeitsdurchlässigen Verbindungen ausgebildet sind, wobei der Prothesenkörper vorzugsweise aus Kunststoff, Metall, Keramik, Glaskeramik, Knochenzement oder einer Kombination daraus gefertigt ist.

Hierdurch wird ein kompakter Aufbau erreicht und der Prothesenkörper gleicht äußerlich bis auf die Öffnungen einem herkömmlichen femoralen Hüftgelenkspacer.

Des Weiteren kann vorgesehen sein, dass die Spülflüssigkeits-Einlassöffnung und die Spülflüssigkeits-Auslassöffnung in einer lateralen Oberfläche des Halses und/oder der distalen Seite des Kugelkopfs angeordnet sind.

Hierdurch kann Zufuhr und Abfuhr der Spülflüssigkeit in den und aus dem Hüftgelenkspacer anatomisch leicht und bequem gelegt werden. An der lateralen seitlichen Oberfläche stören die an die Spülflüssigkeits-Einlassöffnung und die Spülflüssigkeits-Auslassöffnung angeschlossenen Verbindungsmittel beim Laufen besonders wenig und sind auch besonders leicht zugänglich.

Es kann vorgesehen sein, dass der freie Leitungsquerschnitt des zweiseitig offenen Hohlraums der Verankerungshülse gleich groß oder größer ist als der freie Leitungsquerschnitt im Schaft.

Des Weiteren kann vorgesehen sein, dass sich der zweiseitig offene Hohlraum zumindest bereichsweise parallel zur Längsachse des Schafts erstreckt und durch die äußere Mantelfläche des Schafts und der Innenwand der Verankerungshülse begrenzt ist.

Durch diese beiden Maßnahmen wird sichergestellt, dass die Spülflüssigkeit ohne einen störenden Staudruck durch den zweiseitig offenen Hohlraum der Verankerungshülse fließen kann, auch wenn Geweberest mitgeführt werden. Bevorzugt ist hierzu der freie Leitungsquerschnitt des zweiseitig offenen Hohlraums der Verankerungshülse größer als der freie Leitungsquerschnitt im Schaft, besonders bevorzugt zumindest 50% größer als der freie Leitungsquerschnitt im Schaft, ganz besonders bevorzugt zumindest doppelt so groß wie der freie Leitungsquerschnitt im Schaft. Der freie Leitungsquerschnitt im Schaft entspricht dem freien Querschnitt der Leitung für die Spülflüssigkeit im Inneren des Schafts, die zu der zumindest einen Spülflüssigkeitsaustrittsöffnung führt.

Es kann des Weiteren vorgesehen sein, dass die Verankerungshülse eine geschlossene Mantelfläche aufweist oder dass die Verankerungshülse einen Einschnitt aufweist, der parallel zur Längsachse des hohlzylinderförmigen Schafts angeordnet ist.

Hierdurch wird entweder eine vollumfängliche Befestigung ermöglicht oder der Außenumfang der Verankerungshülse lässt sich an den Femurkanal des zu versorgenden Femurs anpassen.

Gemäß einer bevorzugten Ausführung der vorliegenden Erfindung kann vorgesehen sein, dass ein Kragen auf der distalen Seite des Kugelkopfs und distal von der Spülflüssigkeits-Einlassöffnung und der Spülflüssigkeits-Auslassöffnung sowie zwischen dem Kugelkopf und der Verankerungshülse angeordnet ist, wobei der Kragen um das proximale Ende der Verankerungshülse herumläuft, wobei vorzugsweise die zumindest eine Spülflüssigkeitsaustrittsöffnung auf der vom Kragen distal abgewandten Seite am Schaft angeordnet ist.

Durch den Kragen kann verhindert werden, dass eine Knochenzement zur Verankerung des Prothesenkörpers im Femurkanal, eine proximale Öffnung des zweiseitig offenen Hohlraums der Verankerungshülse verschließt oder dessen freien Durchmesser reduziert.

Es kann vorgesehen sein, dass der Kugelkopf mindestens einen flüssigkeitsdurchlässigen Kanal besitzt, der in mindestens einer Spülflüssigkeitsauslassöffnung an der Oberfläche des Kugelkopfs mündet und der mit der Spülflüssigkeits-Einlassöffnung flüssigkeitsdurchlässig verbunden ist.

Ferner kann vorgesehen sein, dass die äußere Mantelfläche der Verankerungshülse eine gummielastische Beschichtung hat.

Auf diese Weise kann eine besonders stabile und/oder anatomisch angepasste Verbindung zum Femur erzeugt werden.

Des Weiteren kann vorgesehen sein, dass die äußere Mantelfläche der Verankerungshülse eine strukturierte Oberfläche zur press-fit Verankerung oder zur Verankerung von Polymethylmethacrylat-Knochenzement aufweist.

Auch auf diese Weise kann eine besonders stabile und/oder anatomisch angepasste Verbindung zum Femur erzeugt werden.

Des Weiteren kann vorgesehen sein, dass die Verankerungshülse sich in distaler Richtung verjüngt, bevorzugt in distaler Richtung konisch zulaufend ist.

Hierdurch ist die Verankerungshülse anatomisch besser an den Femurkanal angepasst. Zudem kann so eine stabilere Verbindung zum Femur erzeugt werden.

Es kann auch vorgesehen sein, dass die Verankerungshülse durch eine separate hohlzylinderförmige gummielastische Hülse umkleidet ist, wobei diese bevorzugt einen Kragen an der proximalen Seite besitzt.

Hierdurch wird eine stabile Verbindung zum Femur ermöglicht. Zudem kann mit dem Kragen verhindert werden, dass der Knochenzementteig zur Verbindung mit dem Femur über die Verankerungshülse hinausfließt und so den zweiseitig offenen Hohlraum auf einer Seite verschließt oder den freien Querschnitt reduziert.

Ferner kann vorgesehen sein, dass eine der zumindest einen Spülflüssigkeitseintrittsöffnung als Spülflüssigkeits-Auslassöffnung ausgebildet ist, wobei hierzu vorzugsweise in der Spülflüssigkeitseintrittsöffnung ein Befestigungsmittel zum lösbaren Verbinden eines röhrenförmigen und flüssigkeitsdurchlässigen Verbindungsmittels zum Abführen der Spülflüssigkeit aus dem Prothesenkörper angeordnet ist, wobei die Spülflüssigkeits-Auslassöffnung nicht durch das angeschlossene Verbindungsmittel verschließbar ist.

Hierdurch kann ein einfacherer Aufbau erreicht werden, der keine oder nur eine einfachere Leitung im Prothesenkörper benötigt. Sofern nur eine Spülflüssigkeitseintrittsöffnung vorhanden ist, kann diese als Sackloch aufgeführt sein, in der das Verbindungsmittel lösbar anschließbar ist.

Bevorzugt kann auch vorgesehen sein, dass der zweiseitig offene Hohlraum der Verankerungshülse innerhalb des Prothesenkörpers flüssigkeitsdurchlässig nicht mit der Spülflüssigkeits-Einlassöffnung verbunden ist und bevorzugt auch nicht mit der Spülflüssigkeits-Auslassöffnung verbunden ist.

Hierdurch wird sichergestellt, dass die Spülung im Bereich des zu behandelnden Gewebes erfolgt und der Hohlraum zum Transfer der Spülflüssigkeit von dem distalen Schaft des Prothesenkörpers zum proximalen Kugelkopf des Prothesenkörpers durch die umlaufende Befestigungsfläche hindurch erfolgen kann.

Der Erfindung liegt die überraschende Erkenntnis zugrunde, dass eine vollständige Spülung der Oberfläche eines Hüftgelenkspacers bei gleichzeitiger vollumfänglicher Verbindung einer Verankerungshülse mit dem Femur um den gesamten Umfang durch einen zweiseitig offenen Hohlraum in einer Verankerungshülse ermöglicht werden kann. Durch den zweiseitig offenen Hohlraum der Hülse kann ein Strom der Spülflüssigkeit durch den zweiseitig offenen Hohlraum geleitet werden, der von einer distalen Seite des Hüftgelenkspacers (am Schaft) bis zu einer proximalen Seite des Hüftgelenkspacers am Kugelkopf geführt werden kann (oder auch umgekehrt). Durch die zumindest eine Spülflüssigkeitseintrittsöffnung und die zumindest eine Spülflüssigkeitsaustrittsöffnung sowie die Spülflüssigkeits-Einlassöffnung und die Spülflüssigkeits-Auslassöffnung wird ein temporärer Hüftgelenkspacer zum kontinuierlichen Spülen einer Kavität im Körper eines Patienten bereitgestellt, indem an der Oberfläche des Prothesenkörpers des Hüftgelenkspacers geeignete Öffnungen und im Inneren des Prothesenkörpers geeignete Leitungen für die Spülflüssigkeit vorhanden sind und indem zwei von außen zugängliche Verbindungsmittel angeschlossen sind oder anschließbar sind, durch die die medizinische Spülflüssigkeit von außen in den Prothesenkörper eingespeist und die verbrauchte Spülflüssigkeit wieder aus dem Prothesenkörper abgeführt werden kann. Bei dem erfindungsgemäßen Hüftgelenkspacer können aufgrund der Verankerungshülse mit dem zweiseitig offenen Hohlraum sowohl eine distale als auch eine proximale Seite des Prothesenkörpers durch einen verbundenen Kreislauf der Spülflüssigkeit gespült werden, ohne dass die Befestigungsfläche des Hüftgelenkspacers, die zur Verbindung mit dem Femur verwendet wird, von der Spülflüssigkeit umflossen werden muss.

Der erfindungsgemäße Hüftgelenkspacer kann vorteilhaft im Rahmen von zweizeitigen septischen Revisionen verwendet werden, bei denen eine Infektion mit zwei oder mehreren mikrobiellen Keimen und insbesondere mit problematischen Keimen vorliegt. Besonders vorteilhaft ist es, dass der Hüftgelenkspacer und das umgebende Weichgewebe und zumindest teilweise auch das umgebende Knochengewebe mit antibiotisch wirksamen Lösungen, wie Antibiotika und auch Antiseptika oder in speziellen Fällen mit Antimykotika, gespült werden können, wobei die Art und die Anzahl der Wirkstoffe und vor allem die Konzentration der antimikrobiellen Wirkstoffe in der Spüllösung (der medizinischen Spülflüssigkeit) exakt eingestellt werden können. Durch Absaugen der Spülflüssigkeit kann auch die Verweildauer der antimikrobiell ausgerüsteten Spülflüssigkeit im Patienten exakt eingestellt werden. Dadurch ist es möglich, über mehrere Tage eine Umspülung der Oberfläche des Hüftgelenkspacers mit exakt zuvor eingestellte Konzentrationen an antimikrobiellen Wirkstoffen in der Spülflüssigkeit zu gewährleisten. Dadurch wird der Schutz vor einer mikrobiellen Wiederbesiedlung der Oberflächen des Hüftgelenkspacers deutlich verringert im Vergleich zu den bisherigen aus antibiotikahaltigem Knochenzement gefertigten Hüftgelenkspacern. Nach der antibiotischen Spülung ist es möglich, die Oberfläche des Hüftgelenkspacers und des umgebenden Gewebes mit Wirkstoff-freien Spülflüssigkeiten zu spülen. Dadurch werden Reste der antimikrobiellen Wirkstoffe entfernt. Eine Resistenzbildung infolge von persistierenden Wirkstoffrückständen ist daher extrem unwahrscheinlich.

Weiterhin ist es vorteilhaft, dass die Spülflüssigkeiten auch solche antimikrobiellen Wirkstoffe enthalten können, die normalerweise nicht in die aus Knochenzement gefertigten Hüftgelenkspacern integriert werden können, weil diese die radikalische Polymerisation des Knochenzementteigs stören beziehungsweise verhindern würden. Exemplarisch dafür seien die Wirkstoffe Rifampicin und Metronidazol genannt.

Wenn die klinischen Parameter erkennen lassen, dass die Infektion beziehungsweise die Entzündung abklingt, dann können die Verbindungsmittel vom Hüftgelenkspacer entfernt werden. Die Verbindungsmittel sind hierzu vorteilhaft mit einem Außengewinde oder über einen Bajonettverschluss oder über einen Steckverschluss mit der Spülflüssigkeits-Einlassöffnung und der Spülflüssigkeits-Auslassöffnung verbunden, beziehungsweise mit jeweils einem zu dem Befestigungselement am Verbindungsmittel passenden Gegenbefestigungselement in oder an der Spülflüssigkeits-Einlassöffnung und der Spülflüssigkeits-Auslassöffnung.

Bevorzugt kann vorgesehen sein, dass die Spülflüssigkeits-Einlassöffnung und die Spülflüssigkeits-Auslassöffnung nach Entfernung der Verbindungsmittel bündig mit der Oberfläche des Hüftgelenkspacers abschließen, um eine Irritation des umgebenden Weichgewebes zu verhindern.

Ein beispielhafter erfindungsgemäßer Hüftgelenkspacer kann zusammengesetzt sein aus
A) einem Kugelkopf mit einer Gleitfläche,
B) einem hohlzylinderförmigen Schaft, der über einen Hals mit dem Kugelkopf verbunden ist,
C) einer Spülflüssigkeits-Einlassöffnung, die unterhalb (distal) des Kugelkopfs angeordnet ist und die mit dem hohlzylinderförmigen Schaft flüssigkeitsdurchlässig verbunden ist,
D) einen ersten Schlauch mit einem ersten Verbindungselement als das erste Verbindungsmittel, wobei die Spülflüssigkeit durch den ersten Schlauch in die Spülflüssigkeits-Einlassöffnung einleitbar ist und das erste Verbindungselement den ersten Schlauch lösbar mit der Spülflüssigkeits-Einlassöffnung verbindet,
E) mindestens einer Spülflüssigkeitsaustrittsöffnung für die Spülflüssigkeit, die an einem distalen Ende des Schafts angeordnet ist,
F) einer Verankerungshülse, die unterhalb des Kugelkopfs und der Spülflüssigkeits-Einlassöffnung angeordnet ist und die den hohlzylinderförmigen Schaft umschließt, wobei die Querschnittsfläche der Verankerungshülse kleiner ist als der Querschnitt des hohlzylinderförmigen Schafts und wobei die Verankerungshülse mit dem Schaft verbunden ist,
G) einem sich parallel zur Längsachse des hohlzylinderförmigen Schafts erstreckenden Hohlraum, der durch die äußeren Mantelfläche des Schafts und der Innenwand der Verankerungshülse begrenzt wird,
H) einem zweiten Schlauch mit einem zweiten Verbindungselement als das zweite Verbindungsmittel, wobei die Spülflüssigkeit durch den zweiten Schlauch aus einer Spülflüssigkeits-Auslassöffnung aus dem Hüftgelenkspacer abführbar ist und das zweite Verbindungselement den zweiten Schlauch lösbar mit der Spülflüssigkeits-Auslassöffnung verbindet.

Der Hüftgelenkspacer kann aus Kunststoff, Metall, Keramik, Glaskeramik und deren Kombinationen gefertigt sein. Vorteilhaft lässt sich der Spacer aus einem Metallkern, der mittels SLM (Selective Laser Melting) hergestellt wurde und einem darum angeordneten Mantel aus Knochenzement fertigen. Es ist auch möglich den gesamten Hüftgelenkspacer aus Metall, wie zum Beispiel Edelstahl und Titanlegierungen, mit SLM zu fertigen. Bevorzugt sind dabei der Edelstahl 1.4404 und die Titanlegierung Ti₆Al₄V.

Im Folgenden werden weitere Ausführungsbeispiele der Erfindung anhand von zwölf schematisch dargestellten Figuren erläutert, ohne jedoch dabei die Erfindung zu beschränken. Dabei zeigt:
Figur 1: eine schematische perspektivische Außenansicht eines beispielhaften ersten erfindungsgemäßen Hüftgelenkspacers mit Spülvorrichtung;
Figur 2: eine schematische perspektivische Seitenansicht des ersten erfindungsgemäßen Hüftgelenkspacers nach Figur 1;
Figur 3: eine schematische perspektivische Außenansicht des ersten erfindungsgemäßen Hüftgelenkspacers nach Figur 1 und 2 auf eine weitere Seite;
Figur 4: eine schematische Querschnittansicht des ersten erfindungsgemäßen Hüftgelenkspacers entsprechend dem Schnitt A in Figur 3;
Figur 5: eine weitere schematische Außenansicht auf den ersten erfindungsgemäßen Hüftgelenkspacer nach den Figuren 1 bis 4;
Figur 6: eine schematische Teilschnittansicht auf einen proximalen Ausschnitt des ersten erfindungsgemäßen Hüftgelenkspacers nach den Figuren 1 bis 5;
Figur 7: eine schematische perspektivische Außenansicht eines beispielhaften zweiten erfindungsgemäßen Hüftgelenkspacers mit Spülvorrichtung;
Figur 8: eine schematische Querschnittansicht des zweiten erfindungsgemäßen Hüftgelenkspacers nach Figur 7;
Figur 9: eine schematische perspektivische Außenansicht eines beispielhaften dritten erfindungsgemäßen Hüftgelenkspacers mit Spülvorrichtung;
Figur 10: eine weitere schematische Außenansicht auf den dritten erfindungsgemäßen Hüftgelenkspacer nach Figur 9;
Figur 11: eine schematische Teilschnittansicht auf den dritten erfindungsgemäßen Hüftgelenkspacer nach den Figuren 9 und 10; und
Figur 12: eine Ausschnittvergrößerung einer schematischen Teilschnittansicht auf einen Teil des Kragens, des Halses und des Kugelkopfs des dritten erfindungsgemäßen Hüftgelenkspacers nach den Figuren 9 bis 11.

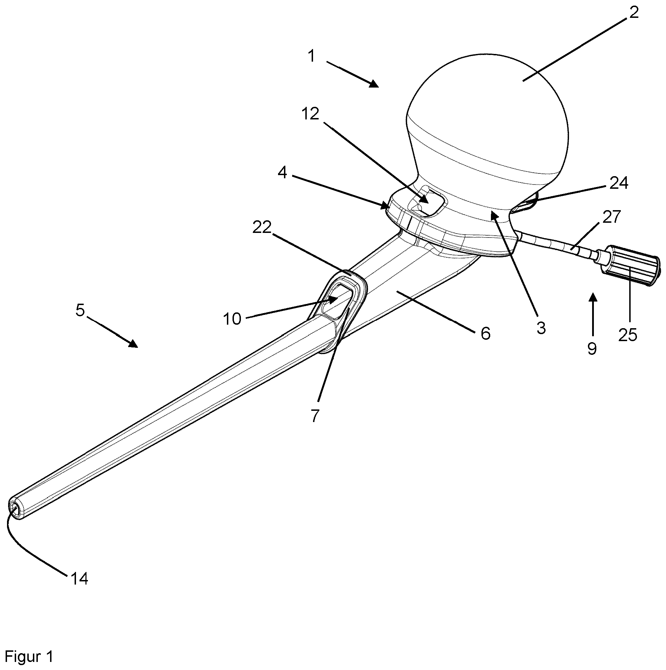

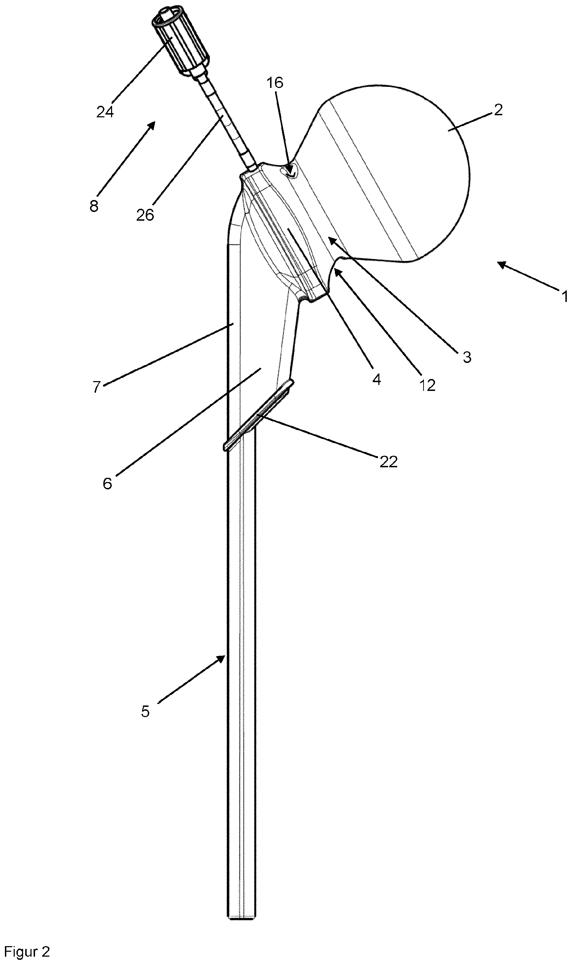

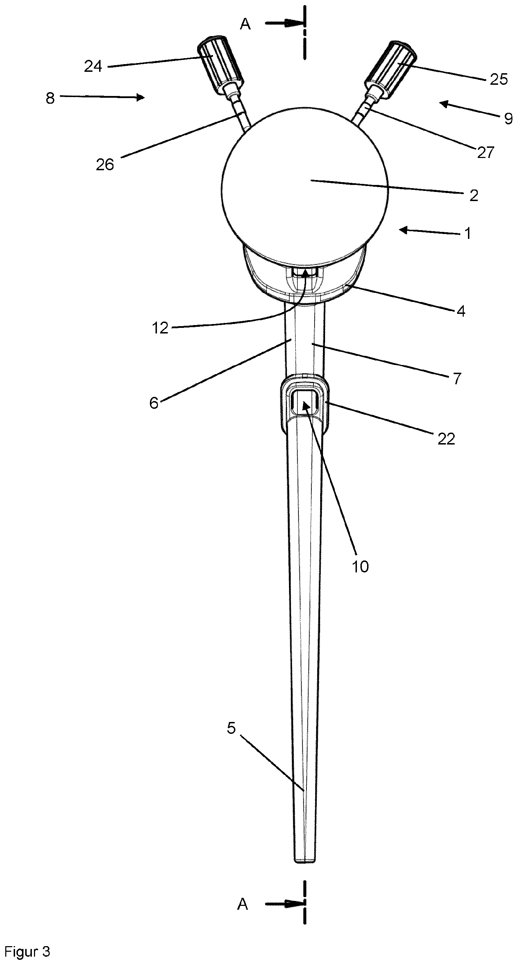

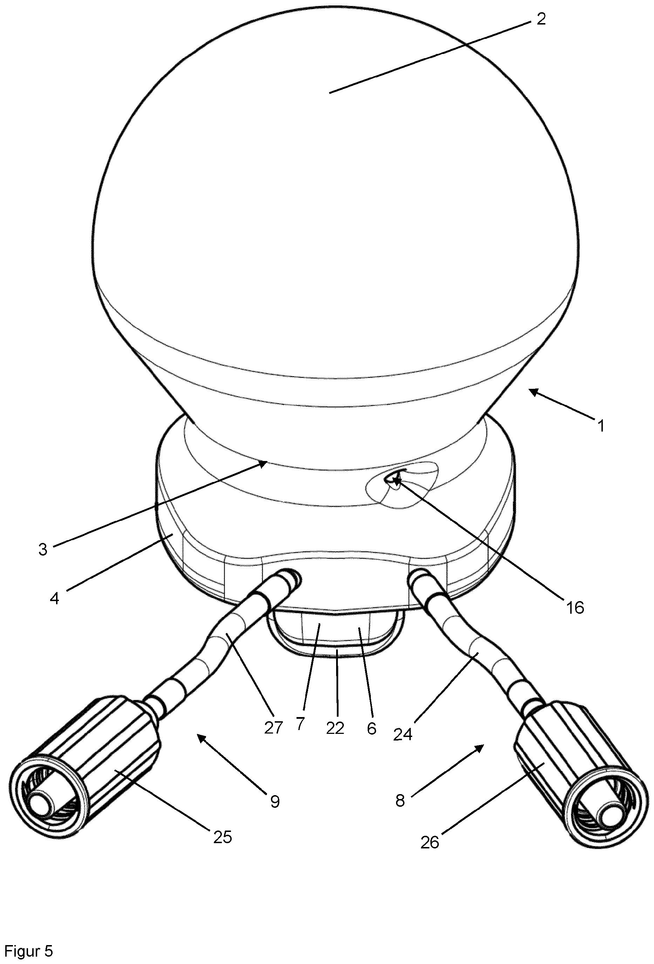

In den Figuren 1 bis 6 sind Abbildungen eines ersten Ausführungsbeispiels eines erfindungsgemäßen Hüftgelenkspacers mit Spülvorrichtung gezeigt. Der femorale Hüftgelenkspacer, das heißt der den Gelenkkopf des Femurs nachbildende und am Femur zu befestigende Hüftgelenkspacer, weist einen Kugelkopf 1 mit einer Gleitfläche 2 an der proximalen Seite auf. Die Gleitfläche 2 kann im eingesetzten Zustand (d.h. im beim Patienten eingesetzten Zustand) an der Hüftgelenkspfanne anliegen und so einen Teil des Hüftgelenks bilden. Der Kugelkopf 1 kann auf der der Gleitfläche 2 gegenüberliegenden distalen Seite über einen Hals 3 mit einem Kragen 4 verbunden sein. Der Hals 3 ist vorzugsweise dünner als der Kugelkopf 1 und der Kragen 4. An der distalen Seite des Kragens 4 kann ein Schaft 5 befestigt sein, der sich in die distale Richtung erstreckt. Zur Befestigung des Hüftgelenkspacers im Femur kann eine umlaufende Befestigungsfläche 6 an einer Verankerungshülse 7 vorgesehen sein, die den Schaft 5 an seiner proximalen Seite umgibt, beziehungsweise umschließt. Mit der umlaufenden Befestigungsfläche 6 kann eine Verbindung des Hüftgelenkspacers in einem Kanal eines Femurs mit Hilfe von Knochenzementteig als "Klebstoff" erfolgen. Der Kugelkopf 1, der Hals 3, der Kragen 4, der Schaft 5 und die Verankerungshülse 7 können einen Prothesenkörper des Hüftgelenkspacers bilden. Der Prothesenkörper entspricht in seiner äußeren Form bis auf die Verankerungshülse 7 weitgehend der äußeren Form bekannter Hüftgelenkspacer.

An einer Seite des ersten beispielhaften Hüftgelenkspacers kann im Unterschied zu bekannten Hüftgelenkspacern ein erstes röhrenförmiges Verbindungsmittel 8 an einer Spülflüssigkeits-Auslassöffnung befestigt und ein zweites röhrenförmiges Verbindungsmittel 9 an einer Spülflüssigkeits-Einlassöffnung befestigt sein. Die Spülflüssigkeits-Einlassöffnung und die Spülflüssigkeits-Auslassöffnung können ins Innere des Prothesenkörpers führen und auf dem Kragen 4 angeordnet sein. Das erste röhrenförmige Verbindungsmittel 8 und das zweite röhrenförmige Verbindungsmittel 9 können flüssigkeitsdurchlässig sein, so dass eine medizinische Spülflüssigkeit durch das zweite röhrenförmige Verbindungsmittel 9 in den Prothesenkörper hineingeleitet werden kann und eine Flüssigkeit durch das erste röhrenförmige Verbindungsmittel 8 aus dem Prothesenkörper abgeführt werden kann. Das erste Verbindungsmittel 8 kann lösbar mit der Spülflüssigkeits-Auslassöffnung verbunden sein und das zweite Verbindungsmittel 9 kann lösbar mit der Spülflüssigkeits-Einlassöffnung verbunden sein.

Die Verankerungshülse 7 kann eine distale Öffnung 10 aufweisen, die in Richtung des distalen Endes des Schafts 5 weist, und eine proximale Öffnung 12 aufweisen, die in Richtung des Kugelkopfs 1 leitet. Die proximale Öffnung 12 kann vom Kragen 4 bis in den Hals 3 reichen.

Am distalen Ende des Schafts 5 kann eine Spülflüssigkeitsaustrittsöffnung 14 angeordnet sein und am Hals 3 kann eine Spülflüssigkeitseintrittsöffnung 16 angeordnet sein. Die Verankerungshülse 7 kann zwischen der Spülflüssigkeitsaustrittsöffnung 14 und der Spülflüssigkeitseintrittsöffnung 16 angeordnet sein.

Die Befestigungsfläche 6 kann an der distalen Seite durch einen umlaufenden Steg 22 und an der proximalen Seite der Befestigungsfläche 6 durch den Kragen 4 begrenzt sein. Der Steg 22 kann sich aus der Oberfläche der Verankerungshülse 7 erheben und die Verankerungshülse 7 an ihrer distalen Seite begrenzen. Der Steg 22 kann als Teil des Prothesenkörpers zu verstehen sein. Durch den vorstehenden Steg 22 und den Kragen 4 kann verhindert werden oder zumindest erschwert werden, dass beim Befestigen des Hüftgelenkspacers am Femur Knochenzementteig außerhalb der Befestigungsfläche 6 vordringt und dadurch die Spülflüssigkeitsaustrittsöffnung 14, die Spülflüssigkeitseintrittsöffnung 16, die Spülflüssigkeits-Einlassöffnung und die Spülflüssigkeits-Auslassöffnung verschließt oder beeinträchtigt beziehungsweise ungewollt das erste Verbindungsmittel 8 oder das zweite Verbindungsmittel 9 am Prothesenkörper festzementiert. Der Kragen 4 kann als ein sich von dem proximalen Ende der Verankerungshülse 7 abstehender Steg ausgeführt sein, so dass die umlaufende Befestigungsfläche 6 auf der proximalen Seite und auf der distalen Seite durch einen vorspringenden Steg begrenzt ist.

Das erste Verbindungsmittel 8 kann einen Luer-Lock-Adapter 24 und einen kurzen, flexiblen Schlauch 26 aufweisen. Ebenso kann das zweite Verbindungsmittel 9 einen Luer-Lock-Adapter 25 und einen kurzen, flexiblen Schlauch 27 aufweisen. Dadurch kann der Hüftgelenkspacer mit dem zweiten Verbindungsmittel 9 über den Luer-Lock-Adapter 25 an eine Quelle für eine medizinische Spülflüssigkeit mit einer Pumpe (nicht gezeigt) angeschlossen werden und das erste Verbindungsmittel 8 über den Luer-Lock-Adapter 24 an einen Sammelbehälter und gegebenenfalls ebenfalls eine Pumpe (nicht gezeigt).

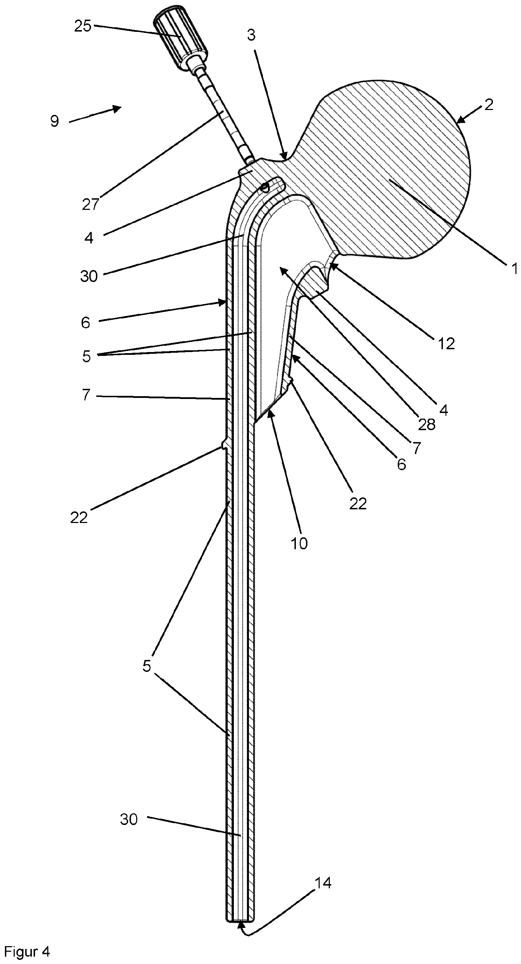

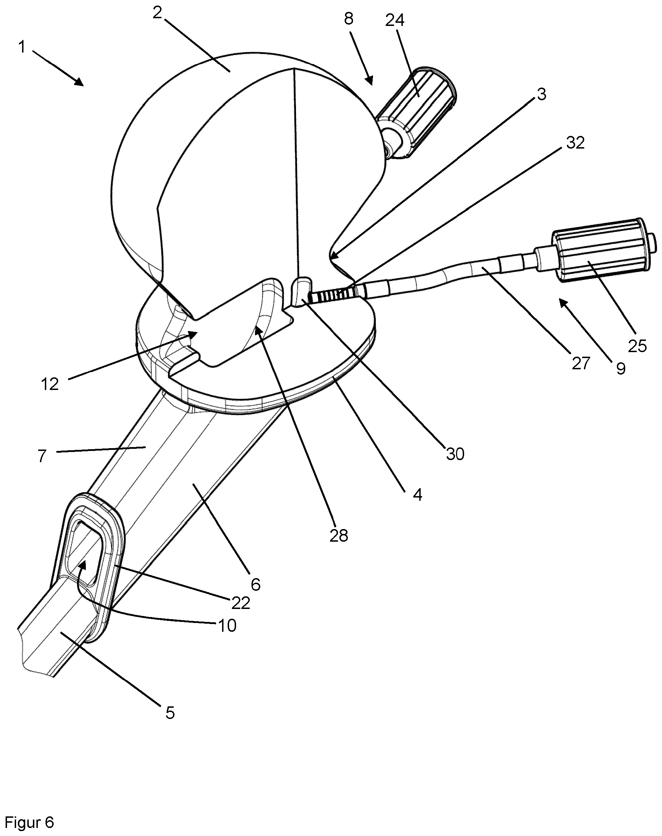

In der Querschnittansicht nach Figur 4 und der Teilschnittansicht nach Figur 6 ist ein Hohlraum 28 zu erkennen, der von den Innenwänden der Verankerungshülse 7 und den Außenwänden des Schafts 5 im Inneren der Verankerungshülse 7 begrenzt sein kann. Der Hohlraum 28 kann die distale Öffnung 10 mit der proximalen Öffnung 12 verbinden. Dadurch kann die Spülflüssigkeit aus der Spülflüssigkeitsaustrittsöffnung 14 herausfließen, anschließend entlang der Oberfläche des Schafts 5 fließen, dann durch die distale Öffnung 10 in den Hohlraum 28, durch den Hohlraum 28 und die proximale Öffnung 12 herausfließen und von dort über die Oberfläche des Halses 3 und des Kugelkopfs 1 zur Spülflüssigkeitseintrittsöffnung 16 fließen. Die gebrauchte Spülflüssigkeit kann anschließend wieder durch die Spülflüssigkeitseintrittsöffnung 16 in den Prothesenkörper eingesaugt werden. Durch die Verankerungshülse 7 mit dem an der distalen Öffnung 10 und der proximalen Öffnung 12 zweiseitig offenen Hohlraum 28 kann die medizinische Spülflüssigkeit also die Oberfläche des Prothesenkörpers sowohl am Schaft 5 als auch am Kugelkopf 1 erreichen. Es reicht dadurch aus, eine Spülflüssigkeitsaustrittsöffnung 14 und eine Spülflüssigkeitseintrittsöffnung 16 vorzusehen, um die überhaupt erreichbaren Oberflächen des Prothesenkörpers mit der medizinischen Spülflüssigkeit erreichen und damit behandeln zu können. Aber auch bei mehr als einer Spülflüssigkeitsaustrittsöffnung 14 und bei mehr als einer Spülflüssigkeitseintrittsöffnung 16 kann über die Verbindung der beiden Seiten über den Hohlraum 28 dafür gesorgt werden, dass ein Flüssigkeitsaustausch zu beiden Seiten des Prothesenkörpers hin möglich ist. Dies verhindert eine Fehlfunktion und ermöglicht eine gleichmäßige Behandlung. Gleichzeitig kann die Verankerungshülse 7 und die Befestigungsfläche 6 vollumfänglich zur Zementierung, das heißt zur Verankerung des Hüftgelenkspacers in einem Kanal eines Femurs verwendet werden und so eine besonders stabile Verbindung zum Femur ermöglichen.

In der Querschnittansicht nach Figur 4 und der Teilschnittansicht nach Figur 6 ist ferner zu erkennen, wie die Spülflüssigkeits-Einlassöffnung mit der Spülflüssigkeitsaustrittsöffnung 14 im Inneren des Prothesenkörpers über eine Leitung 30 im Inneren des Schafts 5 verbunden sein kann. Der Schaft 5 bildet dabei einen Hohlzylinder. Wie insbesondere in Figur 4 gut zu erkennen ist, kann der freie Leitungsquerschnitt des Hohlraums 28 in etwa dreimal größer sein als der freie Leitungsquerschnitt der Leitung 30. Analog kann die Spülflüssigkeitseintrittsöffnung 16 im inneren des Prothesenkörpers über eine separate zweite Leitung (nicht gezeigt) mit der Spülflüssigkeits-Auslassöffnung verbunden sein.

Der Prothesenkörper kann im Wesentlichen aus einem Kunststoff gefertigt sein. Bevorzugt aus einem Knochenzement, wie einem PMMA-Kunststoff, der mit einem Antibiotikum oder mit mehreren Antibiotika beladen sein kann.

Die Leitung 30 kann eine flüssigkeitsdurchlässige Verbindung zwischen der Spülflüssigkeits-Einlassöffnung und der Spülflüssigkeitsaustrittsöffnung 14 herstellen. Die Leitung 30 und die separate zweite Leitung, die die Spülflüssigkeitseintrittsöffnung 16 im inneren des Prothesenkörpers mit der Spülflüssigkeits-Auslassöffnung verbindet, können im Inneren des Prothesenkörpers voneinander getrennt sein, so dass keine Fluidverbindung zwischen der Leitung 30 und der zweiten Leitung im Inneren des Prothesenkörpers besteht.

Am Schlauch 27 des zweiten Verbindungselements 9 kann ein Kupplungselement 32 angeordnet sein, das eine lösbare Verbindung des Schlauchs 27 mit der Spülflüssigkeit-Einlassöffnung ermöglicht. Mit dem Kupplungselement 32 kann eine fluiddichte Verbindung zu der Spülflüssigkeit-Einlassöffnung erzeugt werden.

Unmittelbar vor der Spülflüssigkeits-Auslassöffnung kann in der zweiten Leitung ein Ventilelement (nicht gezeigt) vorgesehen sein, das einen Abfluss von Flüssigkeit aus der zweiten Leitung durch die Spülflüssigkeits-Auslassöffnung aus dem Prothesenkörper in das erste Verbindungsmittel 8 erlaubt und das einen Rückfluss aus dem ersten Verbindungsmittel 8 in die zweite Leitung hinein verhindert. Das erste Verbindungsmittel 8 kann über ein lösbares Verbindungselement mit der Spülflüssigkeits-Auslassöffnung verbunden sein.

Unmittelbar vor der Spülflüssigkeits-Einlassöffnung kann in der Leitung 30 ein Ventilelement (nicht gezeigt) vorgesehen sein, dass einen Zufluss der medizinischen Spülflüssigkeit in die Leitung 30 durch die Spülflüssigkeits-Einlassöffnung in den Prothesenkörper hinein erlaubt und das einen Rückfluss aus der Leitung 30 in das zweite Verbindungsmittel 9 verhindert. Das zweite Verbindungsmittel 9 kann über das Kupplungselement 32 mit der Spülflüssigkeits-Einlassöffnung verbunden sein.

Das erste Verbindungsmittel 8 und das zweite Verbindungsmittel 9 können durch abziehen oder abschrauben von dem Prothesenkörper gelöst werden. Hierzu können flüssigkeitsdurchlässige Gegenbefestigungselemente in den Leitungen 30 im Prothesenkörper vorgesehen sein. Die Gegenbefestigungselemente können beispielsweise durch Hülsen mit Innengewinden realisiert werden, in die das Kupplungselement 32 mit Außengewinde eingeschraubt ist oder einschraubbar ist.

Im eingesetzten Zustand kann der femorale Hüftgelenkspacer wie folgt zum Spülen verwendet werden: Durch das zweite Verbindungsmittel 9 kann eine medizinische Spülflüssigkeit mit einer an die Bedürfnisse des Patienten angepassten Zusammensetzung, wie beispielsweise eine sterile Ringer-Lösung mit einer Mischung geeigneter Antibiotika, in den Prothesenkörper eingespeist werden. Die medizinische Spülflüssigkeit kann durch den Schlauch 27 und durch die Leitung 30 durch den Prothesenkörper fließen und durch die Spülflüssigkeitsaustrittsöffnung 14 am distalen Ende des Schafts 5 aus dem Prothesenkörper austreten. Die Spülflüssigkeit kann anschließend entlang der Oberfläche des Hüftgelenkspacers von der Spülflüssigkeitsaustrittsöffnung 14 durch den Hohlraum 28 in der Verankerungshülse 7 zu der Spülflüssigkeitseintrittsöffnung 16 fließen. Die Bereiche dazwischen können mit einer Schicht der medizinischen Spülflüssigkeit gespült werden. Die gebrauchte Spülflüssigkeit kann an der Spülflüssigkeitseintrittsöffnung 16 wieder in den Prothesenkörper eintreten und durch die separate zweite Leitung zur Spülflüssigkeits-Auslassöffnung fließen. Von dort kann es durch das erste Verbindungsmittel 8 aus dem Prothesenkörper abgesaugt werden und die gebrauchte Spülflüssigkeit anschließend entsorgt oder gesammelt werden.

Wenn keine Spülung mehr erfolgen soll, können die Verbindungsmittel 8, 9 von dem Prothesenkörper getrennt werden und der restliche Hüftgelenkspacer kann wie ein normaler Hüftgelenkspacer auch verwendet werden. Es kann bevorzugt vorgesehen sein, dass sich die Spülflüssigkeits-Einlassöffnung und die Spülflüssigkeits-Auslassöffnung beim Abziehen oder Abschrauben der Verbindungsmittel 8, 9 von dem Prothesenkörper selbsttätig verschließen.

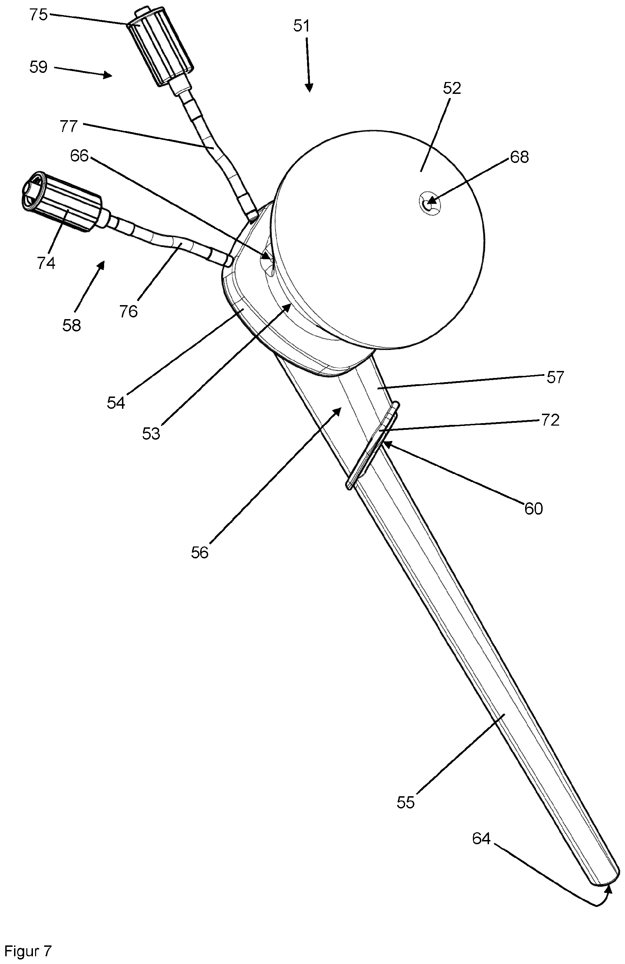

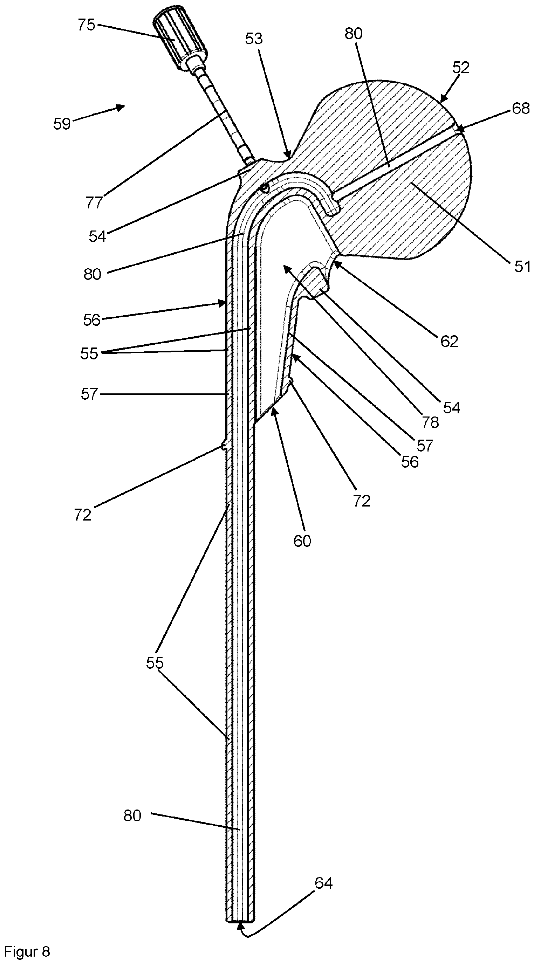

In den Figuren 7 und 8 sind Abbildungen eines zweiten Ausführungsbeispiels eines erfindungsgemäßen Hüftgelenkspacers mit Spülvorrichtung gezeigt. Das zweite Ausführungsbeispiel entspricht sehr weitgehend dem ersten Ausführungsbeispiel. Der femorale Hüftgelenkspacer weist einen Kugelkopf 51 mit einer Gleitfläche 52 an der proximalen Seite auf. Die Gleitfläche 52 kann im eingesetzten Zustand an der Hüftgelenkspfanne anliegen und so einen Teil des Hüftgelenks bilden. Der Kugelkopf 51 kann auf der der Gleitfläche 52 gegenüberliegenden distalen Seite über einen Hals 53 mit einem Kragen 54 verbunden sein. Der Hals 53 ist vorzugsweise dünner als der Kugelkopf 51 und der Kragen 54. An der distalen Seite des Kragens 54 kann ein Schaft 55 befestigt sein, der sich in die distale Richtung erstreckt. Zur Befestigung des Hüftgelenkspacers im Femur kann eine umlaufende Befestigungsfläche 56 an einer Verankerungshülse 57 vorgesehen sein, die den Schaft 55 an seiner proximalen Seite umgibt, beziehungsweise umschließt. Mit der umlaufenden Befestigungsfläche 56 kann eine Verbindung des Hüftgelenkspacers in einem Kanal eines Femurs mit Hilfe von Knochenzementteig als "Klebstoff" erfolgen. Der Kugelkopf 51, der Hals 53, der Kragen 54, der Schaft 55 und die Verankerungshülse 57 können einen Prothesenkörper des Hüftgelenkspacers bilden. Der Prothesenkörper entspricht in seiner äußeren Form bis auf die Verankerungshülse 57 weitgehend der äußeren Form bekannter Hüftgelenkspacer.

An einer Seite des ersten beispielhaften Hüftgelenkspacers kann im Unterschied zu bekannten Hüftgelenkspacern ein erstes röhrenförmiges Verbindungsmittel 58 an einer Spülflüssigkeits-Auslassöffnung befestigt und ein zweites röhrenförmiges Verbindungsmittel 59 an einer Spülflüssigkeits-Einlassöffnung befestigt sein. Die Spülflüssigkeits-Einlassöffnung und die Spülflüssigkeits-Auslassöffnung können ins Innere des Prothesenkörpers führen und am Kragen 54 angeordnet sein. Das erste röhrenförmige Verbindungsmittel 58 und das zweite röhrenförmige Verbindungsmittel 59 können flüssigkeitsdurchlässig sein, so dass eine medizinische Spülflüssigkeit durch das zweite röhrenförmige Verbindungsmittel 59 in den Prothesenkörper hineingeleitet werden kann und eine Flüssigkeit durch das erste röhrenförmige Verbindungsmittel 58 aus dem Prothesenkörper abgeführt werden kann. Das erste Verbindungsmittel 58 kann lösbar mit der Spülflüssigkeits-Auslassöffnung verbunden sein und das zweite Verbindungsmittel 59 kann lösbar mit der Spülflüssigkeits-Einlassöffnung verbunden sein.

Die Verankerungshülse 57 kann eine distale Öffnung 60 aufweisen, die in Richtung des distalen Endes des Schafts 55 weist, und eine proximale Öffnung 62 aufweisen, die in Richtung des Kugelkopfs 51 leitet. Die proximale Öffnung 62 kann vom Kragen 54 bis in den Hals 53 reichen.

Am distalen Ende des Schafts 55 kann eine erste Spülflüssigkeitsaustrittsöffnung 64 angeordnet sein und am Hals 53 kann eine Spülflüssigkeitseintrittsöffnung 66 angeordnet sein. Im Unterschied zum ersten Ausführungsbeispiel nach den Figuren 1 bis 6 ist in der Mitte der Gleitfläche 52 des Kugelkopfs 51 eine zweite Spülflüssigkeitsaustrittsöffnung 68 vorgesehen. Die Verankerungshülse 57 kann zwischen der ersten Spülflüssigkeitsaustrittsöffnung 64 und der Spülflüssigkeitseintrittsöffnung 66 angeordnet sein.

Die Befestigungsfläche 56 kann an der distalen Seite durch einen umlaufenden Steg 72 und an der proximalen Seite der Befestigungsfläche 56 durch den Kragen 54 begrenzt sein. Der Steg 72 kann sich aus der Oberfläche der Verankerungshülse 57 erheben und die Verankerungshülse 57 an ihrer distalen Seite begrenzen. Der Steg 72 kann als Teil des Prothesenkörpers zu verstehen sein. Durch den vorstehenden Steg 72 und den Kragen 54 kann verhindert werden oder zumindest erschwert werden, dass beim Befestigen des Hüftgelenkspacers am Femur Knochenzementteig außerhalb der Befestigungsfläche 56 vordringt und dadurch die erste Spülflüssigkeitsaustrittsöffnung 64, die Spülflüssigkeitseintrittsöffnung 66, die zweite Spülflüssigkeitsaustrittsöffnung 68, die Spülflüssigkeits-Einlassöffnung und die Spülflüssigkeits-Auslassöffnung verschließt oder beeinträchtigt beziehungsweise ungewollt das erste Verbindungsmittel 58 oder das zweite Verbindungsmittel 59 am Prothesenkörper festzementiert. Der Kragen 54 kann als ein sich von dem proximalen Ende der Verankerungshülse 57 abstehender Steg ausgeführt sein, so dass die umlaufende Befestigungsfläche 56 auf der proximalen Seite und auf der distalen Seite durch einen vorspringenden Steg begrenzt ist.

Das erste Verbindungsmittel 58 kann einen Luer-Lock-Adapter 74 und einen kurzen, flexiblen Schlauch 76 aufweisen. Ebenso kann das zweite Verbindungsmittel 59 einen Luer-Lock-Adapter 75 und einen kurzen, flexiblen Schlauch 77 aufweisen. Dadurch kann der Hüftgelenkspacer mit dem zweiten Verbindungsmittel 59 über den Luer-Lock-Adapter 75 an eine Quelle für eine medizinische Spülflüssigkeit mit einer Pumpe (nicht gezeigt) angeschlossen werden und das erste Verbindungsmittel 58 über den Luer-Lock-Adapter 74 an einen Sammelbehälter und gegebenenfalls ebenfalls eine Pumpe (nicht gezeigt).

In der Querschnittansicht nach Figur 8 ist ein Hohlraum 78 zu erkennen, der von den Innenwänden der Verankerungshülse 57 und den Außenwänden des Schafts 55 im Inneren der Verankerungshülse 57 begrenzt sein kann. Der Hohlraum 78 kann die distale Öffnung 60 mit der proximalen Öffnung 62 verbinden. Dadurch kann die Spülflüssigkeit aus der ersten Spülflüssigkeitsaustrittsöffnung 64 herausfließen, anschließend entlang der Oberfläche des Schafts 55 fließen, dann durch die distale Öffnung 60 in den Hohlraum 78, durch den Hohlraum 78 und die proximale Öffnung 62 herausfließen und von dort über die Oberfläche des Halses 53 und des Kugelkopfs 51 zur Spülflüssigkeitseintrittsöffnung 66 fließen. Die gebrauchte Spülflüssigkeit kann anschließend wieder durch die Spülflüssigkeitseintrittsöffnung 66 in den Prothesenkörper eingesaugt werden. Ein zweiter Spülflüssigkeitskreislauf kann zwischen der zweiten Spülflüssigkeitsaustrittsöffnung 68 und der Spülflüssigkeitseintrittsöffnung 66 erzeugt werden. Durch die Verankerungshülse 57 mit dem an der distalen Öffnung 60 und der proximalen Öffnung 62 zweiseitig offenen Hohlraum 78 kann die medizinische Spülflüssigkeit aus der ersten Spülflüssigkeitsaustrittsöffnung 64 am distalen Ende des Schafts 55 also die Oberfläche des Prothesenkörpers sowohl am Schaft 55 als auch am Kugelkopf 51 erreichen. Hierdurch kann über die Verbindung der beiden Seiten über den Hohlraum 78 dafür gesorgt werden, dass ein Flüssigkeitsaustausch zu beiden Seiten des Prothesenkörpers hin möglich ist. Dies verhindert eine Fehlfunktion und ermöglicht eine gleichmäßige Behandlung. Gleichzeitig kann die Verankerungshülse 57 und die Befestigungsfläche 56 vollumfänglich zur Zementierung, das heißt zur Verankerung des Hüftgelenkspacers in einem Kanal eines Femurs verwendet werden und so eine besonders stabile Verbindung zum Femur ermöglichen.

In der Querschnittansicht nach Figur 8 ist ferner zu erkennen, wie die Spülflüssigkeits-Einlassöffnung mit der ersten Spülflüssigkeitsaustrittsöffnung 64 und der zweiten Spülflüssigkeitsaustrittsöffnung 68 im Inneren des Prothesenkörpers über eine Leitung 80 im Inneren des Prothesenkörpers verbunden sein kann. Hierzu ist ein T-Stück vorgesehen, das einen Abzweig von der Spülflüssigkeits-Einlassöffnung zu der ersten Spülflüssigkeitsaustrittsöffnung 64 und der zweiten Spülflüssigkeitsaustrittsöffnung 68 bildet. Der Schaft 55 bildet dabei einen Hohlzylinder. Wie insbesondere in Figur 8 gut zu erkennen ist, kann der freie Leitungsquerschnitt des Hohlraums 78 in etwa dreimal größer sein als der freie Leitungsquerschnitt der Leitung 80. Analog kann die Spülflüssigkeitseintrittsöffnung 66 im inneren des Prothesenkörpers über eine separate zweite Leitung (nicht gezeigt) mit der Spülflüssigkeits-Auslassöffnung verbunden sein.

Der Prothesenkörper kann im Wesentlichen aus einem Kunststoff gefertigt sein. Bevorzugt aus einem Knochenzement, wie einem PMMA-Kunststoff, der mit einem Antibiotikum oder mit mehreren Antibiotika beladen sein kann.

Die Leitung 80 kann eine flüssigkeitsdurchlässige Verbindung zwischen der Spülflüssigkeits-Einlassöffnung und der ersten Spülflüssigkeitsaustrittsöffnung 64 und der zweiten Spülflüssigkeitsaustrittsöffnung 68 herstellen. Die Leitung 80 und die separate zweite Leitung, die die Spülflüssigkeitseintrittsöffnung 66 im inneren des Prothesenkörpers mit der Spülflüssigkeits-Auslassöffnung verbindet, können im Inneren des Prothesenkörpers voneinander getrennt sein, so dass keine Fluidverbindung zwischen der Leitung 80 und der zweiten Leitung im Inneren des Prothesenkörpers besteht.

Am Schlauch 77 des zweiten Verbindungselements 59 kann ein Kupplungselement angeordnet sein, das eine lösbare Verbindung des Schlauchs 77 mit einer Kupplung in der Spülflüssigkeit-Einlassöffnung ermöglicht. Mit dem Kupplungselement kann eine fluiddichte Verbindung zu der Spülflüssigkeit-Einlassöffnung erzeugt werden.

Unmittelbar vor der Spülflüssigkeits-Auslassöffnung kann in der zweiten Leitung ein Ventilelement (nicht gezeigt) vorgesehen sein, das einen Abfluss von Flüssigkeit aus der zweiten Leitung durch die Spülflüssigkeits-Auslassöffnung aus dem Prothesenkörper in das erste Verbindungsmittel 58 erlaubt und das einen Rückfluss aus dem ersten Verbindungsmittel 58 in die zweite Leitung hinein verhindert. Das erste Verbindungsmittel 58 kann über ein lösbares Verbindungselement mit der Spülflüssigkeits-Auslassöffnung verbunden sein.

Unmittelbar vor der Spülflüssigkeits-Einlassöffnung kann in der Leitung 80 ein Ventilelement (nicht gezeigt) vorgesehen sein, dass einen Zufluss der medizinischen Spülflüssigkeit in die Leitung 80 durch die Spülflüssigkeits-Einlassöffnung in den Prothesenkörper hinein erlaubt und das einen Rückfluss aus der Leitung 80 in das zweite Verbindungsmittel 59 verhindert. Das zweite Verbindungsmittel 59 kann über das Kupplungselement mit einer Kupplung in der Spülflüssigkeits-Einlassöffnung verbunden sein.

Das erste Verbindungsmittel 58 und das zweite Verbindungsmittel 59 können durch abziehen oder abschrauben von dem Prothesenkörper gelöst werden. Hierzu können flüssigkeitsdurchlässige Gegenbefestigungselemente in den Leitungen 80 im Prothesenkörper vorgesehen sein. Die Gegenbefestigungselemente können beispielsweise durch Hülsen mit Innengewinden realisiert werden, in die das jeweilige Kupplungselement mit Außengewinde eingeschraubt ist oder einschraubbar ist.

Im eingesetzten Zustand kann der femorale Hüftgelenkspacer wie folgt zum Spülen verwendet werden: Durch das zweite Verbindungsmittel 59 kann eine medizinische Spülflüssigkeit mit einer an die Bedürfnisse des Patienten angepassten Zusammensetzung, wie beispielsweise eine sterile Ringer-Lösung mit einer Mischung geeigneter Antibiotika, in den Prothesenkörper eingespeist werden. Die medizinische Spülflüssigkeit kann durch den Schlauch 77 und durch die Leitung 80 durch den Prothesenkörper fließen und durch die erste Spülflüssigkeitsaustrittsöffnung 64 am distalen Ende des Schafts 55 und durch die zweite Spülflüssigkeitsaustrittsöffnung 68 am proximalen Ende der Gleitfläche 52 aus dem Prothesenkörper austreten. Die Spülflüssigkeit kann anschließend entlang der Oberfläche des Hüftgelenkspacers von der ersten Spülflüssigkeitsaustrittsöffnung 64 durch den Hohlraum 28 in der Verankerungshülse 57 zu der Spülflüssigkeitseintrittsöffnung 66 fließen sowie von der zweiten Spülflüssigkeitsaustrittsöffnung 68 um den Kugelkopf 51 herum zu der Spülflüssigkeitseintrittsöffnung 66 fließen. Die Bereiche dazwischen können mit einer Schicht der medizinischen Spülflüssigkeit gespült werden. Die gebrauchte Spülflüssigkeit kann an der Spülflüssigkeitseintrittsöffnung 66 wieder in den Prothesenkörper eintreten und durch die separate zweite Leitung zur Spülflüssigkeits-Auslassöffnung fließen. Von dort kann es durch das erste Verbindungsmittel 58 aus dem Prothesenkörper abgesaugt werden und die gebrauchte Spülflüssigkeit anschließend entsorgt oder gesammelt werden.

Wenn keine Spülung mehr erfolgen soll, können die Verbindungsmittel 58, 59 von dem Prothesenkörper getrennt werden und der restliche Hüftgelenkspacer kann wie ein normaler Hüftgelenkspacer auch verwendet werden. Es kann bevorzugt vorgesehen sein, dass sich die Spülflüssigkeits-Einlassöffnung und die Spülflüssigkeits-Auslassöffnung beim Abziehen oder Abschrauben der Verbindungsmittel 58, 59 von dem Prothesenkörper selbsttätig verschließen.

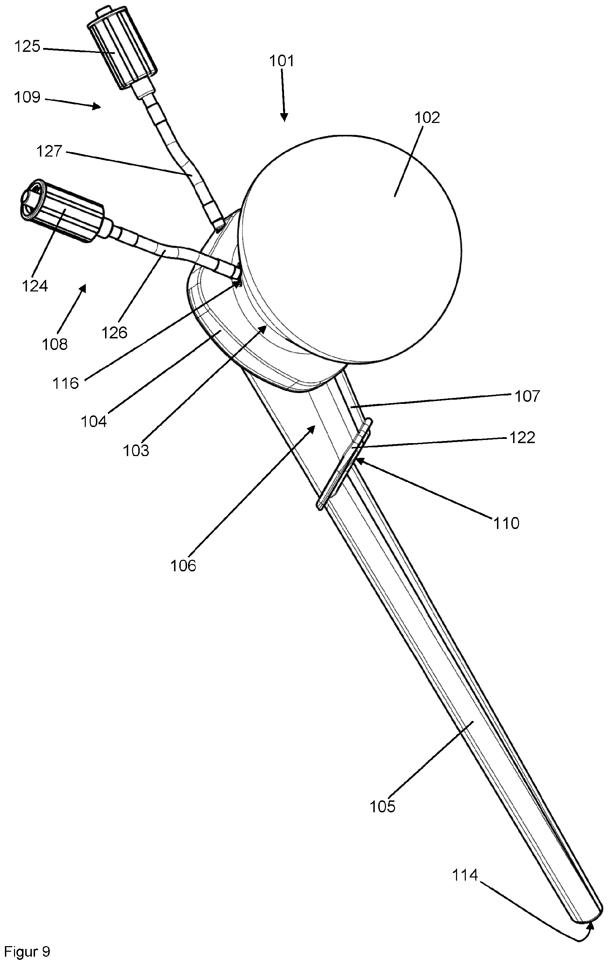

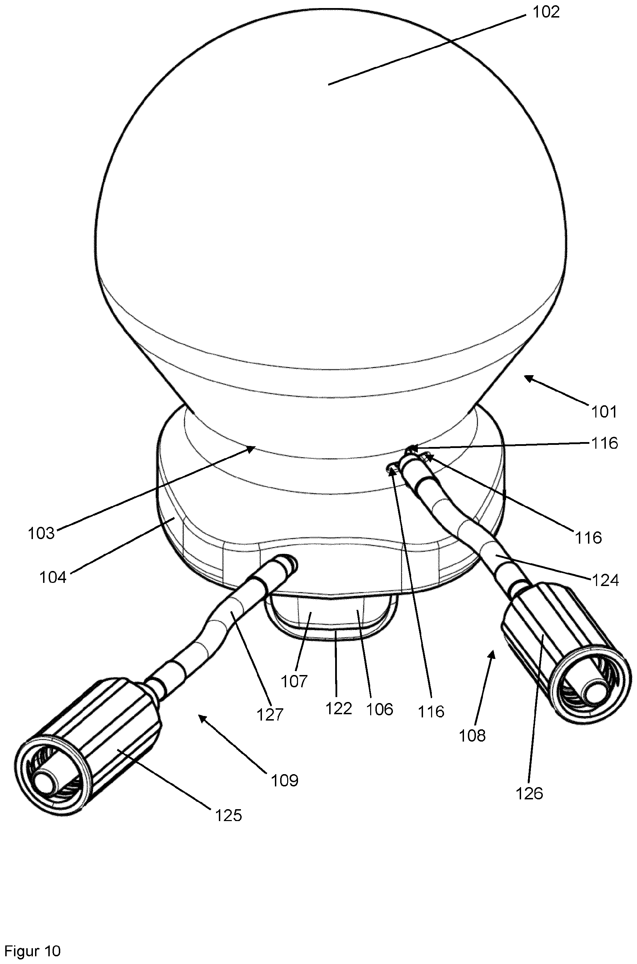

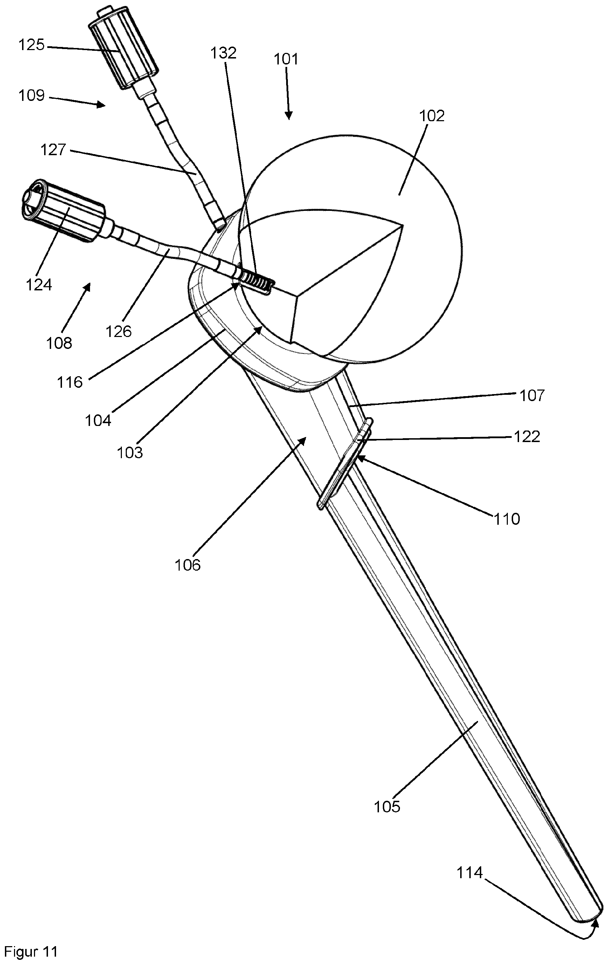

In den Figuren 9 bis 12 sind Abbildungen eines dritten Ausführungsbeispiels eines erfindungsgemäßen Hüftgelenkspacers mit Spülvorrichtung gezeigt. Das dritte Ausführungsbeispiel entspricht sehr weitgehend dem ersten Ausführungsbeispiel. Der femorale Hüftgelenkspacer weist einen Kugelkopf 101 mit einer Gleitfläche 102 an der proximalen Seite auf. Die Gleitfläche 102 kann im eingesetzten Zustand an der Hüftgelenkspfanne anliegen und so einen Teil des Hüftgelenks bilden. Der Kugelkopf 101 kann auf der der Gleitfläche 102 gegenüberliegenden distalen Seite über einen Hals 103 mit einem Kragen 104 verbunden sein. Der Hals 103 ist vorzugsweise dünner als der Kugelkopf 101 und der Kragen 104. An der distalen Seite des Kragens 104 kann ein Schaft 105 befestigt sein, der sich in die distale Richtung erstreckt. Zur Befestigung des Hüftgelenkspacers im Femur kann eine umlaufende Befestigungsfläche 106 an einer Verankerungshülse 107 vorgesehen sein, die den Schaft 105 an seiner proximalen Seite umgibt, beziehungsweise umschließt. Mit der umlaufenden Befestigungsfläche 106 kann eine Verbindung des Hüftgelenkspacers in einem Kanal eines Femurs mit Hilfe von Knochenzementteig als "Klebstoff" erfolgen. Der Kugelkopf 101, der Hals 103, der Kragen 104, der Schaft 105 und die Verankerungshülse 107 können einen Prothesenkörper des Hüftgelenkspacers bilden. Der Prothesenkörper entspricht in seiner äußeren Form bis auf die Verankerungshülse 107 weitgehend der äußeren Form bekannter Hüftgelenkspacer.

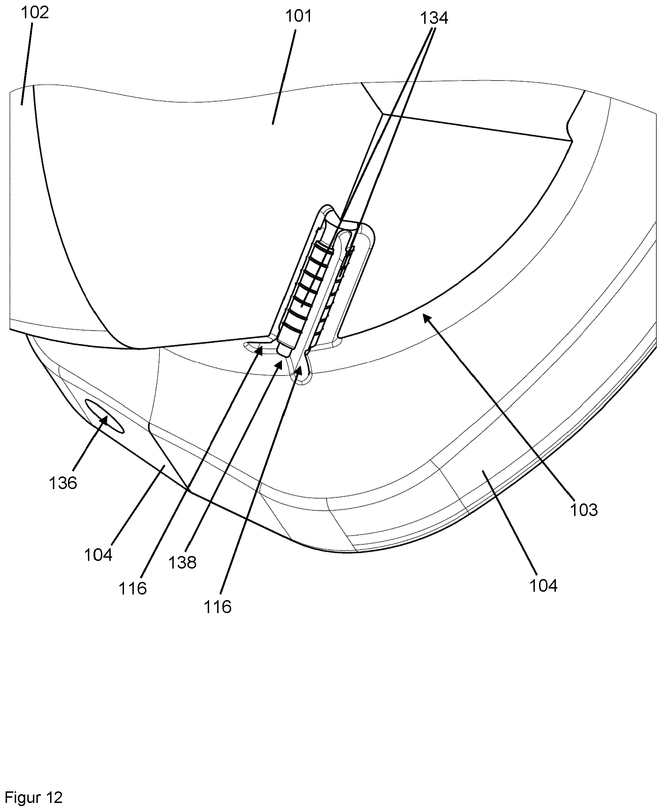

An einer Seite des ersten beispielhaften Hüftgelenkspacers kann im Unterschied zu bekannten Hüftgelenkspacern ein erstes röhrenförmiges Verbindungsmittel 108 an einer Spülflüssigkeits-Auslassöffnung 138 befestigt und ein zweites röhrenförmiges Verbindungsmittel 109 an einer Spülflüssigkeits-Einlassöffnung 136 befestigt sein (siehe Figur 12). Die Spülflüssigkeits-Einlassöffnung 136 kann ins Innere des Prothesenkörpers führen und am Kragen 104 angeordnet sein. Dagegen kann im Unterschied zu den ersten beiden Ausführungsbeispielen die Spülflüssigkeits-Auslassöffnung 138 ein Sackloch im Bereich des Halses 103 sein. In dem Sackloch kann eine Kupplung 134 zur Befestigung eines Kupplungselements 132 an dem ersten Verbindungsmittel 108 vorgesehen sein. Das erste röhrenförmige Verbindungsmittel 108 und das zweite röhrenförmige Verbindungsmittel 109 können flüssigkeitsdurchlässig sein, so dass eine medizinische Spülflüssigkeit durch das zweite röhrenförmige Verbindungsmittel 109 in den Prothesenkörper hineingeleitet werden kann und eine Flüssigkeit durch das erste röhrenförmige Verbindungsmittel 108 aus dem Sackloch abgeführt werden kann. Das erste Verbindungsmittel 108 kann lösbar mit der Spülflüssigkeits-Auslassöffnung 138 verbunden sein und das zweite Verbindungsmittel 109 kann lösbar mit der Spülflüssigkeits-Einlassöffnung 136 verbunden sein.

Die Verankerungshülse 107 kann eine distale Öffnung 110 aufweisen, die in Richtung des distalen Endes des Schafts 105 weist, und eine proximale Öffnung (analog den ersten beiden Ausführungsbeispielen aufweisen, die Richtung des Kugelkopfs 101 leitet. Die proximale Öffnung kann vom Kragen 104 bis in den Hals 103 reichen.

Am distalen Ende des Schafts 105 kann eine Spülflüssigkeitsaustrittsöffnung 114 angeordnet sein und am Hals 103 kann eine Spülflüssigkeitseintrittsöffnung 116 angeordnet sein. Die Spülflüssigkeitseintrittsöffnung 116 ist als dreifache sternförmige Erweiterung der Spülflüssigkeits-Auslassöffnung 138 zu verstehen. Die Spülflüssigkeit wird damit von der Oberfläche des Halses 103 in das gleiche Sackloch eingesaugt aus dem sie auch wieder durch das erste Verbindungsmittel 108 abgesaugt wird, das in der Spülflüssigkeits-Auslassöffnung 138 befestigt ist. Dadurch kann die Ausformung einer Leitung, die die Spülflüssigkeits-Auslassöffnung 138 mit der Spülflüssigkeitseintrittsöffnung 116 verbindet, im Inneren des Prothesenkörpers vermieden werden. Die Verankerungshülse 107 kann zwischen der Spülflüssigkeitsaustrittsöffnung 114 und der Spülflüssigkeitseintrittsöffnung 116 angeordnet sein.

Die Befestigungsfläche 106 kann an der distalen Seite durch einen umlaufenden Steg 122 und an der proximalen Seite der Befestigungsfläche 106 durch den Kragen 104 begrenzt sein. Der Steg 122 kann sich aus der Oberfläche der Verankerungshülse 107 erheben und die Verankerungshülse 107 an ihrer distalen Seite begrenzen. Der Steg 122 kann als Teil des Prothesenkörpers zu verstehen sein. Durch den vorstehenden Steg 122 und den Kragen 104 kann verhindert werden oder zumindest erschwert werden, dass beim Befestigen des Hüftgelenkspacers am Femur Knochenzementteig außerhalb der Befestigungsfläche 106 vordringt und dadurch die Spülflüssigkeitsaustrittsöffnung 114, die Spülflüssigkeitseintrittsöffnung 116, die Spülflüssigkeits-Einlassöffnung 136 und die Spülflüssigkeits-Auslassöffnung 138 verschließt oder beeinträchtigt beziehungsweise ungewollt das erste Verbindungsmittel 108 oder das zweite Verbindungsmittel 109 am Prothesenkörper festzementiert. Der Kragen 104 kann als ein sich von dem proximalen Ende der Verankerungshülse 107 abstehender Steg ausgeführt sein, so dass die umlaufende Befestigungsfläche 106 auf der proximalen Seite und auf der distalen Seite durch einen vorspringenden Steg begrenzt ist.

Das erste Verbindungsmittel 108 kann einen Luer-Lock-Adapter 124 und einen kurzen, flexiblen Schlauch 126 aufweisen. Ebenso kann das zweite Verbindungsmittel 109 einen Luer-Lock-Adapter 125 und einen kurzen, flexiblen Schlauch 127 aufweisen. Dadurch kann der Hüftgelenkspacer mit dem zweiten Verbindungsmittel 109 über den Luer-Lock-Adapter 125 an eine Quelle für eine medizinische Spülflüssigkeit mit einer Pumpe (nicht gezeigt) angeschlossen werden und das erste Verbindungsmittel 108 über den Luer-Lock-Adapter 124 an einen Sammelbehälter und gegebenenfalls ebenfalls eine Pumpe (nicht gezeigt).

Der innere Aufbau des dritten Ausführungsbeispiels entspricht dem in Figur 4 gezeigten Querschnitt des ersten Ausführungsbeispiels. Es kann daher im inneren der Verankerungshülse 107 ein Hohlraum vorgesehen sein (der dem Hohlraum 28 nach dem ersten Ausführungsbeispiel (siehe Figur 4) entspricht), der von den Innenwänden der Verankerungshülse 107 und den Außenwänden des Schafts 105 im Inneren der Verankerungshülse 107 begrenzt sein kann. Der Hohlraum kann die distale Öffnung 110 mit der proximalen Öffnung verbinden. Dadurch kann die Spülflüssigkeit aus der Spülflüssigkeitsaustrittsöffnung 114 herausfließen, anschließend entlang der Oberfläche des Schafts 105 fließen, dann durch die distale Öffnung 110 in den Hohlraum, durch den Hohlraum und die proximale Öffnung herausfließen und von dort über die Oberfläche des Halses 103 und des Kugelkopfs 101 zur Spülflüssigkeitseintrittsöffnung 116 fließen. Die gebrauchte Spülflüssigkeit kann anschließend wieder durch die Spülflüssigkeitseintrittsöffnung 116 in den Prothesenkörper eingesaugt werden. Durch die Verankerungshülse 107 mit dem an der distalen Öffnung 110 und der proximalen Öffnung zweiseitig offenen Hohlraum kann die medizinische Spülflüssigkeit also die Oberfläche des Prothesenkörpers sowohl am Schaft 105 als auch am Kugelkopf 101 erreichen. Es reicht dadurch aus, eine Spülflüssigkeitsaustrittsöffnung 114 und eine Spülflüssigkeitseintrittsöffnung 116 vorzusehen, um die überhaupt erreichbaren Oberflächen des Prothesenkörpers mit der medizinischen Spülflüssigkeit erreichen und damit behandeln zu können. Aber auch bei mehr als einer Spülflüssigkeitsaustrittsöffnung 114 und bei mehr als einer Spülflüssigkeitseintrittsöffnung 116 kann über die Verbindung der beiden Seiten über den Hohlraum dafür gesorgt werden, dass ein Flüssigkeitsaustausch zu beiden Seiten des Prothesenkörpers hin möglich ist. Dies verhindert eine Fehlfunktion und ermöglicht eine gleichmäßige Behandlung. Gleichzeitig kann die Verankerungshülse 107 und die Befestigungsfläche 106 vollumfänglich zur Zementierung, das heißt zur Verankerung des Hüftgelenkspacers in einem Kanal eines Femurs verwendet werden und so eine besonders stabile Verbindung zum Femur ermöglichen.

Analog der Querschnittansicht nach Figur 4 kann die Spülflüssigkeits-Einlassöffnung 136 mit der Spülflüssigkeitsaustrittsöffnung 114 im Inneren des Prothesenkörpers über eine Leitung im Inneren des Schafts 105 verbunden sein. Der Schaft 105 bildet dabei einen Hohlzylinder. Der freie Leitungsquerschnitt des Hohlraums kann in etwa größer sein als, insbesondere zumindest doppelt so groß sein wie, der freie Leitungsquerschnitt der Leitung.

Der Prothesenkörper kann im Wesentlichen aus einem Kunststoff gefertigt sein. Bevorzugt aus einem Knochenzement, wie einem PMMA-Kunststoff, der mit einem Antibiotikum oder mit mehreren Antibiotika beladen sein kann.

Die Leitung kann eine flüssigkeitsdurchlässige Verbindung zwischen der Spülflüssigkeits-Einlassöffnung 136 und der Spülflüssigkeitsaustrittsöffnung 114 herstellen. Die Leitung kann im Inneren des Prothesenkörpers von dem Sackloch der Spülflüssigkeits-Auslassöffnung 138 getrennt sein, so dass keine Fluidverbindung zwischen der Leitung und der Spülflüssigkeits-Auslassöffnung 138 im Inneren des Prothesenkörpers besteht.

Am Schlauch 126 des ersten Verbindungselements 108 kann das Kupplungselement 132 angeordnet sein, das eine lösbare Verbindung des Schlauchs 126 mit der Spülflüssigkeit-Auslassöffnung 138 ermöglicht. Mit dem Kupplungselement 132 kann eine fluiddichte Verbindung zu der Spülflüssigkeit-Einlassöffnung 138 erzeugt werden.

Unmittelbar vor der Spülflüssigkeits-Einlassöffnung 136 kann in der Leitung ein Ventilelement (nicht gezeigt) vorgesehen sein, dass einen Zufluss der medizinischen Spülflüssigkeit in die Leitung durch die Spülflüssigkeits-Einlassöffnung 136 in den Prothesenkörper hinein erlaubt und das einen Rückfluss aus der Leitung in das zweite Verbindungsmittel 109 verhindert. Das erste Verbindungsmittel 108 kann über das Kupplungselement 132 mit der Spülflüssigkeits-Einlassöffnung 136 verbunden sein.

Das erste Verbindungsmittel 108 und das zweite Verbindungsmittel 109 können durch abziehen oder abschrauben von dem Prothesenkörper gelöst werden. Hierzu können flüssigkeitsdurchlässige Gegenbefestigungselemente im Prothesenkörper vorgesehen sein. Die Gegenbefestigungselemente können beispielsweise durch Hülsen mit Innengewinden als die Kupplung 134 realisiert werden, in die das Kupplungselement 132 mit Außengewinde eingeschraubt ist oder einschraubbar ist.

Im eingesetzten Zustand kann der femorale Hüftgelenkspacer wie folgt zum Spülen verwendet werden: Durch das zweite Verbindungsmittel 109 kann eine medizinische Spülflüssigkeit mit einer an die Bedürfnisse des Patienten angepassten Zusammensetzung, wie beispielsweise eine sterile Ringer-Lösung mit einer Mischung geeigneter Antibiotika, in den Prothesenkörper eingespeist werden. Die medizinische Spülflüssigkeit kann durch den Schlauch 127 und durch die Leitung durch den Prothesenkörper fließen und durch die Spülflüssigkeitsaustrittsöffnung 114 am distalen Ende des Schafts 105 aus dem Prothesenkörper austreten. Die Spülflüssigkeit kann anschließend entlang der Oberfläche des Hüftgelenkspacers von der Spülflüssigkeitsaustrittsöffnung 114 durch den Hohlraum in der Verankerungshülse 107 zu der Spülflüssigkeitseintrittsöffnung 116 fließen. Die Bereiche dazwischen können mit einer Schicht der medizinischen Spülflüssigkeit gespült werden. Die gebrauchte Spülflüssigkeit kann an der Spülflüssigkeitseintrittsöffnung 116 wieder in den Prothesenkörper eintreten und durch die Spülflüssigkeits-Auslassöffnung 138 und durch das erste Verbindungsmittel 108 aus dem Prothesenkörper abgesaugt werden und die gebrauchte Spülflüssigkeit anschließend entsorgt oder gesammelt werden.

Wenn keine Spülung mehr erfolgen soll, können die Verbindungsmittel 108, 109 von dem Prothesenkörper getrennt werden und der restliche Hüftgelenkspacer kann wie ein normaler Hüftgelenkspacer auch verwendet werden. Es kann bevorzugt vorgesehen sein, dass sich die Spülflüssigkeits-Einlassöffnung und die Spülflüssigkeits-Auslassöffnung beim Abziehen oder Abschrauben der Verbindungsmittel 108, 109 von dem Prothesenkörper selbsttätig verschließen.

Der Schaft 5, 55, 105 und der Kugelkopf 1, 51, 101 können gemäß einer alternativen Ausführungsform über eine Gewindestange mit Außengewinde aneinandergeschraubt werden.

Die Verwendung von einem mit Antibiotika oder Antimykotika oder anderer pharmazeutisch wirksamer Substanzen versetztem PMMA zumindest als äußere Schicht erfindungsgemäßer Hüftgelenkspacer hat den Vorteil, dass initial großflächig eine besonders große Menge der Wirkstoffe zur Verfügung steht. Zudem ergibt sich ein besonderer kombinatorischer Effekt, dass nämlich der Kreislauf der medizinischen Spülflüssigkeit die Freisetzung der Wirkstoffe an der Oberfläche des Hüftgelenkspacers fördert und verstärkt.

### Bezugszeichenliste

- 1, 51, 101: Kugelkopf
- 2, 52, 102: Gleitfläche
- 3, 53, 103: Hals
- 4, 54, 104: Kragen / Steg
- 5, 55, 105: Schaft
- 6, 56, 106: Befestigungsfläche
- 7, 57, 107: Verankerungshülse
- 8, 58, 108: Verbindungsmittel
- 9, 59, 109: Verbindungsmittel
- 10, 60, 110: Distale Öffnung
- 12, 62: Proximale Öffnung
- 14, 64, 114: Spülflüssigkeitsaustrittsöffnung
- 16, 66, 116: Spülflüssigkeitseintrittsöffnung
- 22, 72, 122: Steg
- 24, 74, 124: Luer-Lock-Adapter
- 25, 75, 125: Luer-Lock-Adapter
- 26, 76, 126: Schlauch
- 27, 77, 127: Schlauch
- 28, 78: Hohlraum
- 30, 80: Leitung
- 32, 132: Kupplungselement
- 68: Spülflüssigkeitsaustrittsöffnung
- 134: Kupplung
- 136: Spülflüssigkeits-Einlassöffnung
- 138: Spülflüssigkeits-Auslassöffnung

## Patentansprüche

1. Femoraler Hüftgelenkspacer zum zeitweisen Ersetzen eines Teils eines Hüftgelenks, der Hüftgelenkspacer aufweisend
einen Prothesenkörper, der Prothesenkörper aufweisend einen Kugelkopf (1, 51, 101) mit einer Gleitfläche (2, 52, 102), einen Hals (3, 53, 103), der an seiner proximalen Seite mit dem Kugelkopf (1, 51, 101) verbunden ist, einen Schaft (5, 55, 105), der auf einer dem Kugelkopf (1, 51, 101) gegenüberliegenden distalen Seite des Halses (3, 53, 103) mit dem Hals (3, 53, 103) verbunden ist, und eine Verankerungshülse (7, 57, 107), die den Schaft (5, 55, 105) auf einer proximalen Seite des Schafts (5, 55, 105) mit einer umlaufenden Befestigungsfläche (6, 56, 106) umschließt und die mit dem Schaft (5, 55, 105) verbunden ist,
der Hüftgelenkspacer ferner aufweisend eine Spülflüssigkeits-Einlassöffnung (136) in einer Oberfläche des Prothesenkörpers,
eine Spülflüssigkeits-Auslassöffnung (138) in der Oberfläche des Prothesenkörpers, zumindest eine Spülflüssigkeitsaustrittsöffnung (14, 64, 114) auf einer distalen Seite des Schafts (5, 55, 105) und zumindest eine Spülflüssigkeitseintrittsöffnung (16, 66, 116) am Kugelkopf (1, 51, 101) oder am Hals (3, 53, 103), wobei
die zumindest eine Spülflüssigkeitsaustrittsöffnung (14, 64, 114) im Inneren des Prothesenkörpers mit der Spülflüssigkeits-Einlassöffnung (136) flüssigkeitsdurchlässig verbunden ist und nicht flüssigkeitsdurchlässig mit der Spülflüssigkeits-Auslassöffnung (138) verbunden ist und
die zumindest eine Spülflüssigkeitseintrittsöffnung (16, 66, 116) im Inneren des Prothesenkörpers mit der Spülflüssigkeits-Auslassöffnung (138) flüssigkeitsdurchlässig verbunden ist und nicht flüssigkeitsdurchlässig mit der Spülflüssigkeits-Einlassöffnung (136) verbunden ist und wobei
im Inneren der Verankerungshülse (7, 57, 107) ein zweiseitig offener Hohlraum (28, 78) ausgebildet ist, der eine proximale Seite der Verankerungshülse (7, 57, 107) mit einer distalen Seite der Verankerungshülse (7, 57, 107) flüssigkeitsdurchlässig verbindet.

2. Hüftgelenkspacer nach Anspruch 1, **dadurch gekennzeichnet, dass**
der Hüftgelenkspacer ein erstes röhrenförmiges und flüssigkeitsdurchlässiges Verbindungsmittel (8, 58, 108) zum Abführen der Spülflüssigkeit aus dem Prothesenkörper aufweist, wobei das erste Verbindungsmittel (8, 58, 108) mit der Spülflüssigkeits-Auslassöffnung (138) flüssigkeitsdurchlässig verbunden oder verbindbar ist, und
der Hüftgelenkspacer ein zweites röhrenförmiges und flüssigkeitsdurchlässiges Verbindungsmittel (9, 59, 109) zum Zuführen einer medizinischen Spülflüssigkeit in den Prothesenkörper aufweist, wobei das zweite Verbindungsmittel (9, 59, 109) mit der Spülflüssigkeits-Einlassöffnung (136) flüssigkeitsdurchlässig verbunden oder verbindbar ist.

3. Hüftgelenkspacer nach Anspruch 1 oder 2, **dadurch gekennzeichnet, dass**
die zumindest eine Spülflüssigkeitsaustrittsöffnung (14, 64, 114) und die zumindest eine Spülflüssigkeitseintrittsöffnung (16, 66, 116) in der Oberfläche des Prothesenkörpers außerhalb der umlaufenden Befestigungsfläche (6, 56, 106) angeordnet sind.

4. Hüftgelenkspacer nach einem der vorangehenden Ansprüche, **dadurch gekennzeichnet, dass**
die Spülflüssigkeits-Einlassöffnung (136) und/oder die Spülflüssigkeits-Auslassöffnung (138) am Kugelkopf (1, 51, 101) oder am Hals (3, 53, 103) des Prothesenkörpers angeordnet sind, wobei vorzugsweise die Spülflüssigkeits-Einlassöffnung (136) und/oder die Spülflüssigkeits-Auslassöffnung (138) an einer lateralen Seite des Halses (3, 53, 103) des Prothesenkörpers angeordnet sind.

5. Hüftgelenkspacer nach einem der vorangehenden Ansprüche, **dadurch gekennzeichnet, dass**
der Schaft (5, 55, 105) hohlzylinderförmig ist, wobei eine Leitung (30, 80) im Inneren des Schafts (5, 55, 105) ausgebildet ist, die die zumindest eine Spülflüssigkeitsaustrittsöffnung (14, 64, 114) mit der Spülflüssigkeits-Einlassöffnung (136) flüssigkeitsdurchlässig verbindet.

6. Hüftgelenkspacer nach einem der vorangehenden Ansprüche, **dadurch gekennzeichnet, dass**
die Befestigungsfläche (6, 56, 106) begrenzt ist, wobei die Befestigungsfläche (6, 56, 106) zur Aufnahme von Knochenzementteig geeignet ist, oder
die Befestigungsfläche (6, 56, 106) mit zwei umlaufenden, sich aus der Oberfläche des Prothesenkörpers erhebenden Stegen (22, 72, 122) an einer proximalen Seite und an einer distalen Seite der Verankerungshülse (7, 57, 107) begrenzt ist, wobei die Befestigungsfläche (6, 56, 106) zur Aufnahme von Knochenzementteig innerhalb der Stege (22, 72, 122) geeignet ist, oder
die Befestigungsfläche (6, 56, 106) mit einem umlaufenden, sich aus der Oberfläche des Prothesenkörpers erhebenden Steg (22, 72, 122) an einer distalen Seite der Verankerungshülse (7, 57, 107) und einem umlaufenden, sich aus der Oberfläche des Prothesenkörpers erhebenden Kragen (4, 54, 104) an einer proximalen Seite der Verankerungshülse (7, 57, 107) begrenzt ist, wobei die Befestigungsfläche (6, 56, 106) zur Aufnahme von Knochenzementteig innerhalb des Stegs (22, 72, 122) und des Kragens (4, 54, 104) geeignet ist.

7. Hüftgelenkspacer nach einem der vorangehenden Ansprüche, **dadurch gekennzeichnet, dass**
eine erste Spülflüssigkeitsaustrittsöffnung (14, 64, 114) der wenigstens einen Spülflüssigkeitsaustrittsöffnung (14, 64, 114) am distalen Ende des Schafts (5, 55, 105) angeordnet ist, wobei
vorzugsweise eine zweite Spülflüssigkeitsaustrittsöffnung (68) auf der proximalen Seite des Kugelkopfs (1, 51, 101) angeordnet ist, wobei die eine erste Spülflüssigkeitsaustrittsöffnung (14, 64, 114) und die zweite Spülflüssigkeitsaustrittsöffnung (68) im Inneren des Prothesenkörpers mit der Spülflüssigkeits-Einlassöffnung (136) flüssigkeitsdurchlässig verbunden sind.

8. Hüftgelenkspacer nach einem der vorangehenden Ansprüche, **dadurch gekennzeichnet, dass**
die zumindest eine Spülflüssigkeitseintrittsöffnung (16, 66, 116) am Hals (3, 53, 103) des Prothesenkörpers und/oder an der distalen Seite des Kugelkopfs (1, 51, 101) angeordnet ist, und/oder
die Verankerungshülse (7, 57, 107) zwischen der zumindest einen Spülflüssigkeitsaustrittsöffnung (14, 64, 114) und der zumindest einen Spülflüssigkeitseintrittsöffnung (16, 66, 116) angeordnet ist, so dass die Spülflüssigkeit bei einer flüssigkeitsdichten Verbindung der umlaufenden Befestigungsfläche (6, 56, 106) mit einem umgebenden Femurkanal nur durch den zweiseitig offenen Hohlraum (28, 78) von der zumindest einen Spülflüssigkeitsaustrittsöffnung (14, 64, 114) zu der zumindest einen Spülflüssigkeitseintrittsöffnung (16, 66, 116) fließen kann.

9. Hüftgelenkspacer nach einem der vorangehenden Ansprüche, **dadurch gekennzeichnet, dass**
an der Spülflüssigkeits-Einlassöffnung (136) im Inneren des Prothesenkörpers oder an der Oberfläche des Prothesenkörpers eine selbstdichtende Kupplung angeordnet ist und an der Spülflüssigkeits-Auslassöffnung (138) im Inneren des Prothesenkörpers oder an der Oberfläche des Prothesenkörpers eine selbstdichtende Kupplung (134) angeordnet ist, wobei vorzugsweise Verbindungsmittel (8, 9, 58, 59, 108, 109) lösbar mit der Spülflüssigkeits-Einlassöffnung (136) und mit der Spülflüssigkeits-Auslassöffnung (138) verbunden oder verbindbar sind.

10. Hüftgelenkspacer nach einem der vorangehenden Ansprüche, **dadurch gekennzeichnet, dass**
die Summe der Querschnittsflächen aller der zumindest einen Spülflüssigkeitseintrittsöffnung (16, 66, 116) zusammen zumindest genauso groß ist wie die Querschnittsfläche der Spülflüssigkeits-Einlassöffnung (136) und/oder die Summe der Querschnittsflächen aller der zumindest einen Spülflüssigkeitsaustrittsöffnung (14, 64, 114) zumindest genauso groß ist wie die Querschnittsfläche der Spülflüssigkeits-Auslassöffnung (138).

11. Hüftgelenkspacer nach einem der vorangehenden Ansprüche, **dadurch gekennzeichnet, dass**
in einer ersten Leitung innerhalb des Prothesenkörpers, die die zumindest eine Spülflüssigkeitseintrittsöffnung (16, 66, 116) mit der Spülflüssigkeits-Auslassöffnung (138) flüssigkeitsdurchlässig verbindet, ein erstes Ventil angeordnet ist, das nur durch Anlegen eines Unterdrucks an der Spülflüssigkeits-Auslassöffnung (138) zu öffnen ist und das ein Zurückfließen der Spülflüssigkeit in die erste Leitung verhindert, und/oder in einer zweiten Leitung (30, 80) innerhalb des Prothesenkörpers, die die zumindest eine Spülflüssigkeitsaustrittsöffnung (14, 64, 114) mit der Spülflüssigkeits-Einlassöffnung (136) flüssigkeitsdurchlässig verbindet, ein zweites Ventil angeordnet ist, das nur durch Anlegen eines Unterdrucks an der Spülflüssigkeits-Einlassöffnung (136) zu öffnen ist und das ein Zurückfließen der Spülflüssigkeit in die zweite Leitung (30, 80) verhindert.

12. Hüftgelenkspacer nach einem der vorangehenden Ansprüche, **dadurch gekennzeichnet, dass**
der freie Leitungsquerschnitt des zweiseitig offenen Hohlraums (28, 78) der Verankerungshülse (7, 57, 107) gleich groß oder größer ist als der freie Leitungsquerschnitt im Schaft (5, 55, 105) und/oder sich der zweiseitig offene Hohlraum (28, 78) zumindest bereichsweise parallel zur Längsachse des Schafts (5, 55, 105) erstreckt und durch die äußere Mantelfläche des Schafts (5, 55, 105) und der Innenwand der Verankerungshülse (7, 57, 107) begrenzt ist.

13. Hüftgelenkspacer nach einem der vorangehenden Ansprüche, **dadurch gekennzeichnet, dass**
die Verankerungshülse (7, 57, 107) eine geschlossene Mantelfläche aufweist oder dass die Verankerungshülse (7, 57, 107) einen Einschnitt aufweist, der parallel zur Längsachse des hohlzylinderförmigen Schafts (5, 55, 105) angeordnet ist, und/oder ein Kragen (4, 54, 104) auf der distalen Seite des Kugelkopfs (1, 51, 101) und distal von der Spülflüssigkeits-Einlassöffnung (136) und der Spülflüssigkeits-Auslassöffnung (138) sowie zwischen dem Kugelkopf (1, 51, 101) und der Verankerungshülse (7, 57, 107) angeordnet ist, wobei der Kragen (4, 54, 104) um das proximale Ende der Verankerungshülse (7, 57, 107) herumläuft, wobei vorzugsweise die zumindest eine Spülflüssigkeitsaustrittsöffnung (14, 64, 114) auf der vom Kragen (4, 54, 104) distal abgewandten Seite am Schaft (5, 55, 105) angeordnet ist.

14. Hüftgelenkspacer nach einem der vorangehenden Ansprüche, **dadurch gekennzeichnet, dass**
die äußere Mantelfläche der Verankerungshülse (7, 57, 107) eine gummielastische Beschichtung hat und/oder die äußere Mantelfläche der Verankerungshülse (7, 57, 107) eine strukturierte Oberfläche zur press-fit Verankerung oder zur Verankerung von Polymethylmethacrylat-Knochenzement aufweist, und/oder
die Verankerungshülse (7, 57, 107) sich in distaler Richtung verjüngt, bevorzugt in distaler Richtung konisch zulaufend ist, und/oder
die Verankerungshülse (7, 57, 107) durch eine separate hohlzylinderförmige gummielastische Hülse umkleidet ist, wobei diese bevorzugt einen Kragen (4, 54, 104) an der proximalen Seite besitzt.

15. Hüftgelenkspacer nach einem der vorangehenden Ansprüche, **dadurch gekennzeichnet, dass**
eine der zumindest einen Spülflüssigkeitseintrittsöffnung (16, 66, 116) als Spülflüssigkeits-Auslassöffnung (138) ausgebildet ist, wobei hierzu vorzugsweise in der Spülflüssigkeitseintrittsöffnung (16, 66, 116) ein Befestigungsmittel zum lösbaren Verbinden eines röhrenförmigen und flüssigkeitsdurchlässigen Verbindungsmittels zum Abführen der Spülflüssigkeit aus dem Prothesenkörper angeordnet ist, wobei die Spülflüssigkeits-Auslassöffnung (138) nicht durch das angeschlossene Verbindungsmittel verschließbar ist.

16. Hüftgelenkspacer nach einem der vorangehenden Ansprüche, **dadurch gekennzeichnet, dass**
der zweiseitig offene Hohlraum (28, 78) der Verankerungshülse (7, 57, 107) innerhalb des Prothesenkörpers flüssigkeitsdurchlässig nicht mit der Spülflüssigkeits-Einlassöffnung (136) verbunden ist und bevorzugt auch nicht mit der Spülflüssigkeits-Auslassöffnung (138) verbunden ist.

## Claims

1. A femoral hip joint spacer for temporary replacement of a part of a hip joint, the hip joint spacer having
a prosthesis body, the prosthesis body having a ball head (1, 51, 101) with a sliding surface (2, 52, 102), a neck (3, 53, 103), the proximal side of which is connected to the ball head (1, 51, 101), a stem (5, 55, 105) which is connected to the neck (3, 53, 103) on a distal side of the neck (3, 53, 103) which is opposite the ball head (1, 51, 101), and an anchoring sleeve (7, 57, 107) which encloses the stem (5, 55, 105) on a proximal side of the stem (5, 55, 105) with a circumferential fastening area (6, 56, 106) and which is connected to the stem (5, 55, 105),
the hip joint spacer further having an irrigation liquid inlet opening (136) in a surface of the prothesis body,
an irrigation liquid outlet opening (138) in the surface of the prosthesis body,
at least one irrigation liquid discharge opening (14, 64, 114) on a distal side of the stem (5, 55, 105) and at least one irrigation liquid intake opening (16, 66, 116) on the ball head (1, 51, 101) or on the neck (3, 53, 103), wherein
the at least one irrigation liquid discharge opening (14, 64, 114) is connected inside the prothesis body in a liquid-permeable manner to the irrigation liquid inlet opening (136) and is not connected inside the prothesis body in a liquid-permeable manner to the irrigation liquid outlet opening (138), and
the at least one irrigation liquid intake opening (16, 66, 116) is connected inside the prothesis body in a liquid-permeable manner to the irrigation liquid outlet opening (138) and is not connected inside the prothesis body in a liquid-permeable manner to the irrigation liquid inlet opening (136), and wherein
a cavity (28, 78) which is open on two sides is formed inside the anchoring sleeve (7, 57, 107), which cavity connects a proximal side of the anchoring sleeve (7, 57, 107) to a distal side of the anchoring sleeve (7, 57, 107) in a liquid-permeable manner.

2. The hip joint spacer according to Claim 1, **characterized in that**
the hip joint spacer has a first tubular and liquid-permeable connecting means (8, 58, 108) for draining the irrigation liquid from the prosthesis body, wherein the first connecting means (8, 58, 108) is connected or connectable in a liquid-permeable manner to the irrigation liquid outlet opening (138), and
the hip joint spacer has a second tubular and liquid-permeable connecting means (9, 59, 109) for feeding a medical irrigation liquid into the prosthesis body, wherein the second connecting means (9, 59, 109) is connected or connectable in a liquid-permeable manner to the irrigation liquid inlet opening (136).

3. The hip joint spacer according to Claim 1 or 2, **characterized in that**
the at least one irrigation liquid discharge opening (14, 64, 114) and the at least one irrigation liquid intake opening (16, 66, 116) are arranged in the surface of the prothesis body outside the circumferential fastening area (6, 56, 106).

4. The hip joint spacer according to any one of the preceding claims, **characterized in that** the irrigation liquid inlet opening (136) and/or the irrigation liquid outlet opening (138) is/are arranged on the ball head (1, 51, 101) or on the neck (3, 53, 103) of the prothesis body, wherein the irrigation liquid inlet opening (136) and/or the irrigation liquid outlet opening (138) is/are preferably arranged on a lateral side of the neck (3, 53, 103) of the prothesis body.

5. The hip joint spacer according to any one of the preceding claims, **characterized in that** the stem (5, 55, 105) is hollow cylinder-shaped, wherein a duct (30, 80) is formed inside the stem (5, 55, 105) which connects the at least one irrigation liquid discharge opening (14, 64, 114) to the irrigation liquid inlet opening (136) in a liquid-permeable manner.

6. The hip joint spacer according to any one of the preceding claims, **characterized in that** the fastening area (6, 56, 106) is delimited, wherein the fastening area (6, 56, 106) is suitable for accommodating bone cement paste, or
the fastening area (6, 56, 106) is delimited by two circumferential crosspieces (22, 72, 122) extending up out of the surface of the prothesis body on a proximal side and on a distal side of the anchoring sleeve (7, 57, 107), wherein the fastening area (6, 56, 106) is suitable for accommodating bone cement paste within the crosspieces (22, 72, 122), or
the fastening area (6, 56, 106) is delimited by one circumferential crosspiece (22, 72, 122) extending up out of the surface of the prothesis body on a distal side of the anchoring sleeve (7, 57, 107) and one circumferential collar (4, 54, 104) extending up out of the surface of the prothesis body on a proximal side of the anchoring sleeve (7, 57, 107), wherein the fastening area (6, 56, 106) is suitable for accommodating bone cement paste within the crosspiece (22, 72, 122) and the collar (4, 54, 104).

7. The hip joint spacer according to any one of the preceding claims, **characterized in that** a first irrigation liquid discharge opening (14, 64, 114) of the at least one irrigation liquid discharge opening (14, 64, 114) is arranged at the distal end of the stem (5, 55, 105), wherein
a second irrigation liquid discharge opening (68) is preferably arranged on the proximal side of the ball head (1, 51, 101), wherein the one first irrigation liquid discharge opening (14, 64, 114) and the second irrigation liquid discharge opening (68) inside the prothesis body are connected to the irrigation liquid inlet opening (136) in a liquid-permeable manner.

8. The hip joint spacer according to any one of the preceding claims, **characterized in that** the at least one irrigation liquid intake opening (16, 66, 116) is arranged on the neck (3, 53, 103) of the prothesis body and/or on the distal side of the ball head (1, 51, 101), and/or
the anchoring sleeve (7, 57, 107) is arranged between the at least one irrigation liquid discharge opening (14, 64, 114) and the at least one irrigation liquid intake opening (16, 66, 116) so that, when the circumferential fastening area (6, 56, 106) is connected in a liquid-tight manner to a surrounding femoral canal, the irrigation liquid may only flow through the cavity (28, 78) which is open on two sides from the at least one irrigation liquid discharge opening (14, 64, 114) to the at least one irrigation liquid intake opening (16, 66, 116).

9. The hip joint spacer according to any one of the preceding claims, **characterized in that** a self-sealing coupling is arranged at the irrigation liquid inlet opening (136) inside the prothesis body or on the surface of the prosthesis body, and a self-sealing coupling (134) is arranged at the irrigation liquid outlet opening (138) inside the prothesis body or on the surface of the prothesis body, wherein connecting means (8, 9, 58, 59, 108, 109) are preferably detachably connected or connectable to the irrigation liquid inlet opening (136) and to the irrigation liquid outlet opening (138).

10. The hip joint spacer according to any one of the preceding claims, **characterized in that** the sum of the cross-sectional areas of all of the at least one irrigation liquid intake opening (16, 66, 116) together is at least as great as the cross-sectional area of the irrigation liquid inlet opening (136), and/or
the sum of the cross-sectional areas of all of the at least one irrigation liquid discharge opening (14, 64, 114) is at least as great as the cross-sectional area of the irrigation liquid outlet opening (138).

11. The hip joint spacer according to any one of the preceding claims, **characterized in that** a first valve is arranged in a first duct within the prothesis body, which connects the at least one irrigation liquid intake opening (16, 66, 116) to the irrigation liquid outlet opening (138) in a liquid-permeable manner, the first valve being openable solely by applying a vacuum at the irrigation liquid outlet opening (138) and preventing backflow of the irrigation liquid into the first duct, and/or
a second valve is arranged in a second duct (30, 80) within the prothesis body, which connects the at least one irrigation liquid discharge opening (14, 64, 114) to the irrigation liquid inlet opening (136) in a liquid-permeable manner, the second valve being openable solely by applying a vacuum at the irrigation liquid inlet opening (136) and preventing backflow of the irrigation liquid into the second duct (30, 80).

12. The hip joint spacer according to any one of the preceding claims, **characterized in that** the free duct cross-section of the cavity (28, 78) of the anchoring sleeve (7, 57, 107), which is open on two sides, is as large as or larger than the free duct cross-section in the stem (5, 55, 105), and/or the cavity (28, 78) which is open on two sides extends at least in certain areas parallel to the longitudinal axis of the stem (5, 55, 105) and is delimited by the outer lateral surface of the stem (5, 55, 105) and the internal wall of the anchoring sleeve (7, 57, 107).

13. The hip joint spacer according to any one of the preceding claims, **characterized in that** the anchoring sleeve (7, 57, 107) has a closed lateral surface or the anchoring sleeve (7, 57, 107) has a notch which is arranged parallel to the longitudinal axis of the hollow cylinder-shaped stem (5, 55, 105), and/or
a collar (4, 54, 104) is arranged on the distal side of the ball head (1, 51, 101) and distally from the irrigation liquid inlet opening (136) and the irrigation liquid outlet opening (138) as well as between the ball head (1, 51, 101) and the anchoring sleeve (7, 57, 107), wherein the collar (4, 54, 104) runs around the proximal end of the anchoring sleeve (7, 57, 107), wherein the at least one irrigation liquid discharge opening (14, 64, 114) is preferably arranged on the side facing distally away from the collar (4, 54, 104) on the stem (5, 55, 105).

14. The hip joint spacer according to any one of the preceding claims, **characterized in that** the outer lateral surface of the anchoring sleeve (7, 57, 107) has a rubbery-elastic coating and/or the outer lateral surface of the anchoring sleeve (7, 57, 107) has a structured surface for press-fit anchoring or for anchoring polymethyl methacrylate bone cement, and/or
the anchoring sleeve (7, 57, 107) tapers in the distal direction, preferably converges conically in the distal direction, and/or
the anchoring sleeve (7, 57, 107) is sheathed by a separate hollow cylinder-shaped rubbery elastic sleeve, wherein said sleeve preferably has a collar (4, 54, 104) on the proximal side.

15. The hip joint spacer according to any one of the preceding claims, **characterized in that** one of the at least one irrigation liquid intake opening (16, 66, 116) is formed as an irrigation liquid outlet opening (138), wherein, to this end, a fastening means for detachably connecting a tubular and liquid-permeable connecting means for draining the irrigation liquid from the prothesis body is preferably arranged in the irrigation liquid intake opening (16, 66, 116), wherein the irrigation liquid outlet opening (138) cannot be closed by the connected connecting means.

16. The hip joint spacer according to any one of the preceding claims, **characterized in that** the cavity (28, 78) of the anchoring sleeve (7, 57, 107), which is open on two sides, is within the prothesis body not connected in a liquid-permeable manner to the irrigation liquid inlet opening (136) and preferably is within the prothesis body also not connected to the irrigation liquid outlet opening (138).

## Revendications

1. Entretoise de l'articulation fémorale de la hanche permettant d'insérer par intermittence une partie d'une articulation de la hanche, l'entretoise de l'articulation de hanche présentant
un corps de prothèse, le corps de prothèse présentant une tête sphérique (1, 51, 101) dotée d'une surface de glissement (2, 52, 102), un col (3, 53, 103) qui est relié avec la tête sphérique (1, 51, 101) au niveau de son côté proximal, une tige (5, 55, 105) qui est reliée avec le col (3, 53, 13) sur un côté du col (3, 53, 103) distal situé en face de la tête sphérique (1, 51, 101), et un manchon d'ancrage (7, 57, 107) qui entoure la tige (5, 55, 105) sur un côté proximal de la tige (5, 55, 105) avec une surface de fixation (6, 56, 106) périphérique,
l'entretoise de l'articulation de la hanche présentant en outre un orifice d'entrée de liquide de lavage (136) dans une surface du corps de prothèse,
un orifice de sortie de liquide de lavage (138) dans la surface du corps de prothèse, au moins un orifice d'évacuation de liquide de lavage (14, 64, 114) sur un côté distal de la tige (5, 55, 105) et au moins un orifice d'entrée de liquide de lavage (16, 66, 116) sur la tête sphérique (1, 51, 101) ou sur le col (3, 53, 103), où
l'au moins un orifice d'évacuation de liquide de lavage (14, 64, 114) est relié à l'intérieur du corps de prothèse avec l'orifice d'entrée de liquide de lavage (136) en laissant passer le liquide et est relié avec l'orifice de sortie de liquide de lavage (138) en ne laissant pas passer le liquide, et
l'au moins un orifice d'entrée de liquide de lavage (16, 66, 116) est relié à l'intérieur du corps de prothèse avec l'orifice de sortie de liquide de lavage (138) en laissant passer le liquide et est relié avec l'orifice d'entrée de liquide de lavage (136) en ne laissant pas passer de liquide, et où
un espace creux (28, 78) ouvert de deux côtés est formé à l'intérieur du manchon d'ancrage (7, 57, 107), qui relie un côté proximal du manchon d'ancrage (7, 57, 107) avec un côté distal du manchon d'ancrage (7, 57, 107) en laissant passer le liquide.

2. Entretoise d'articulation de la hanche selon la revendication 1, **caractérisée en ce que** l'entretoise d'articulation de la hanche présente une première pièce de liaison (8, 58, 108) en forme de tube et laissant passer les liquides, pour évacuer le liquide de lavage du corps de prothèse, où la première pièce de liaison (8, 58, 108) est reliée ou peut être reliée avec l'orifice de sortie de liquide de lavage (138) en laissant passer le liquide, et
l'entretoise d'articulation de la hanche présente une deuxième pièce de liaison (9, 59, 109) en forme de tube et laissant passer le liquide pour amener un liquide de lavage médicinal dans le corps de prothèse, où la deuxième pièce de liaison (9, 59, 109) est reliée ou peut être reliée avec l'orifice d'entrée de liquide de lavage (136) en laissant passer le liquide.

3. Entretoise d'articulation de la hanche selon la revendication 1 ou la revendication 2, **caractérisée en ce que**
l'au moins un orifice d'évacuation de liquide de lavage (14, 64, 114) et l'au moins un orifice d'entrée de liquide de lavage (16, 66, 116) sont disposés sur la surface du corps de prothèse en dehors de la surface de fixation (6, 56, 106) périphérique.

4. Entretoise d'articulation de la hanche selon l'une des revendications précédentes, **caractérisée en ce que**
l'orifice d'entrée de liquide de lavage (136) et/ou l'orifice de sortie de liquide de lavage (138) sont disposés sur la tête sphérique (1, 51, 101) ou sur le col (3, 53, 103) du corps de prothèse, où, de préférence, l'orifice d'entrée de liquide de lavage (136) et/ou l'orifice de sortie de liquide de lavage (138) sont disposés sur un côté latéral du col (3, 53, 103) du corps de prothèse.

5. Entretoise d'articulation de la hanche selon l'une des revendications précédentes, **caractérisée en ce que**
la tige (55, 55, 105) est en forme de cylindre creux, où la conduite (30, 80) est aménagée à l'intérieur de la tige (5, 55, 105) qui relie l'au moins un orifice d'évacuation de liquide de lavage (14, 64, 114) avec l'orifice d'entrée de liquide de lavage (136) en laissant passer le liquide.

6. Entretoise d'articulation de la hanche selon l'une des revendications précédentes, **caractérisée en ce que**
la surface de fixation (6, 56, 106) est délimitée, où la surface de fixation (6, 56, 106) est appropriée pour la réception d'une pâte de ciment osseux, ou
la surface de fixation (6, 56, 106) est délimitée avec deux passages (22, 72, 122) s'élevant hors de la surface du corps de prothèse au niveau d'un côté proximal et est délimitée à un côté distal du manchon d'ancrage (7, 57, 107), où la surface de fixation (6, 56, 106) est appropriée pour la réception d'une pâte de ciment osseux à l'intérieur des passages (22, 72, 122), ou
la surface de fixation (6, 56, 106) est délimitée avec un passage (22, 72, 122) périphérique s'élevant hors de la surface du corps de prothèse au niveau d'un côté distal du manchon d'ancrage (7, 57, 107), et avec une collerette (4, 54, 104) périphérique s'élevant hors de la surface du corps de prothèse au niveau d'un côté proximal du manchon d'ancrage (7, 57, 107), où la surface de fixation (6, 56, 106) est appropriée pour la réception de pâte de ciment osseux à l'intérieur du passage (22, 72, 122) et de la collerette (4, 54, 104).

7. Entretoise d'articulation de la hanche selon l'une des revendications précédentes, **caractérisée en ce**
**qu'**un premier orifice d'évacuation de liquide de lavage (14, 64, 114) parmi l'au moins un orifice d'évacuation de liquide de lavage (14, 64, 114) est disposé à l'extrémité distale de la tige (5, 55, 105), où
de préférence un deuxième orifice d'évacuation de liquide de lavage (68) est disposé sur un côté proximal de la tête sphérique (1, 51, 101), où le premier orifice d'évacuation de liquide de lavage (14, 64, 114) et le deuxième orifice d'évacuation de liquide de lavage (68) sont reliés en laissant passer le liquide à l'intérieur du corps de prothèse avec l'orifice d'entrée de liquide de lavage (136).

8. Entretoise d'articulation de la hanche selon l'une des revendications précédentes, **caractérisée en ce que**
l'au moins un orifice d'entrée de liquide de lavage (16, 66, 116) est disposé sur le col (3, 53, 103) du corps de prothèse et/ou sur le côté distal de la tête sphérique (1, 51, 101), et/ou
le manchon d'ancrage (7, 57, 107) est disposé entre l'au moins un orifice d'évacuation de liquide de lavage (14, 64, 114) et l'au moins un orifice d'entrée de liquide de lavage (16, 66, 116) de sorte que le liquide peut s'écouler, lors d'une liaison étanche aux liquides de la surface de fixation (6, 56, 106) périphérique avec un canal du fémur l'entourant uniquement par un espace creux (28, 78) ouvert de deux côtés, de l'au moins un orifice d'évacuation de liquide de lavage (14, 64, 114) vers l'au moins un orifice d'entrée de liquide de lavage (16, 66, 116).

9. Entretoise d'articulation de la hanche selon l'une des revendications précédentes, **caractérisée en ce**
**qu'**un accouplement auto-étanche est disposé sur l'orifice d'entrée de liquide de lavage (136) à l'intérieur du corps de prothèse ou sur la surface du corps de prothèse et qu'un accouplement (134) auto-étanche est disposé sur l'orifice de sortie de liquide de lavage (138) à 'intérieur du corps de prothèse ou sur la surface du corps de prothèse, où, de préférence, des systèmes de fixation (8, 9, 58, 59, 108, 109) sont reliés ou peuvent être reliés de manière amovible avec l'orifice d'entrée de liquide de lavage (136) et avec l'orifice de sortie de liquide de lavage (138).

10. Entretoise d'articulation de la hanche selon l'une des revendications précédentes, **caractérisée en ce que**
la somme des surfaces transversales de tous les orifices parmi l'au moins un orifice d'entrée de liquide de lavage (16, 66, 116) est dans l'ensemble aussi grande que la surface transversale de l'orifice d'entrée de liquide de lavage (136), et/ou la somme des surfaces transversales de tous les orifices parmi l'au moins un orifice d'évacuation de liquide de lavage (14, 64, 114) est dans l'ensemble aussi grande que la surface transversale de l'orifice de sortie de liquide de lavage (138).

11. Entretoise d'articulation de la hanche selon l'une des revendications précédentes, **caractérisée en ce**
**que**, dans une première conduite à l'intérieur du corps de prothèse, qui relie l'au moins un orifice d'entrée de liquide de lavage (16, 66, 116) avec l'orifice de sortie de liquide de lavage -138) en laissant passer le liquide, une première vanne est disposée, qui n'est ouverte que pour appliquer une dépression à l'orifice de sortie de liquide de lavage (138) et qui empêche un reflux du liquide de lavage dans la première conduite, et/ou
**que**, dans une deuxième conduite (30, 80) à l'intérieur du corps de prothèse qui relie l'au moins un orifice d'évacuation de liquide de lavage (14, 64, 114) avec l'orifice d'entrée de liquide de lavage (136) en laissant passer le liquide, une deuxième vanne est disposée, qui n'est ouverte que pour appliquer une dépression à l'orifice d'entrée de liquide de lavage (136) et qui empêche un reflux du liquide de lavage dans la deuxième conduite (30, 80).

12. Entretoise d'articulation de la hanche selon l'une des revendications précédentes, **caractérisée en ce que**
la section transversale de conduite libre de l'espace creux (28, 78) ouvert des deux côtés du manchon d'ancrage (7, 57, 107) est de la même taille ou est plus grande que la section transversale de conduite libre dans la tige (5, 55, 105), et/ou l'espace creux (28, 78) ouvert des deux côtés s'étend au moins par endroits parallèlement par rapport à l'axe longitudinal de la tige (5, 55, 105) et est délimité par la surface d'enveloppe extérieure de la tige (5, 55, 105) et la paroi intérieure du manchon d'ancrage (7, 57, 107).

13. Entretoise d'articulation de la hanche selon l'une des revendications précédentes, **caractérisée en ce que**
le manchon d'ancrage (7, 57, 107) présente une surface d'enveloppe fermée ou que le manchon d'ancrage (7, 57, 107) présente une incision qui est disposée parallèlement par rapport à l'axe longitudinal de la tige (5, 55, 105) en forme de cylindre creux, et/ou une collerette (4, 54, 104) est disposée sur le côté distal de la tête sphérique (1, 51, 101) et de manière distale à partir de l'orifice d'entrée de liquide de lavage (136) et de l'orifice de sortie de liquide de lavage (138) ainsi qu'entre la tête sphérique (1, 51, 101) et le manchon d'ancrage (7, 57, 107), où la collerette (4, 54, 104) s'enroule autour de l'extrémité proximale du manchon d'ancrage (7, 57, 107), où, de préférence, l'au moins un orifice d'évacuation de liquide de lavage (14, 64, 114) est disposé sur le côté opposé de manière distale à la collerette (4, 54, 104) sur la tige (5, 55, 105).

14. Entretoise d'articulation de la hanche selon l'une des revendications précédentes, **caractérisée en ce que**
la surface d'enveloppe extérieure du manchon d'ancrage (7, 57, 107) a un revêtement viscoélastique et/ou la surface d'enveloppe extérieure du manchon d'ancrage (7, 57, 107) présente une surface structurée pour un ancrage de type press-fit ou pour l'ancrage d'un ciment osseux de polyméthyl méthacrylate, et/ou
le manchon d'ancrage (7, 57, 107) s'amenuise en direction distale, de préférence, est jointif de manière conique en direction distale, et/ou
le manchon d'ancrage (7, 57, 107) est revêtu d'un manchon viscoélastique en forme de cylindre creux, où celui-ci possède de préférence une collerette (4, 54, 104) sur le côté proximal.

15. Entretoise d'articulation de la hanche selon l'une des revendications précédentes, **caractérisée en ce**
**qu'**au moins un orifice d'entrée de liquide de lavage (16, 66, 116) est formé en tant qu'orifice de sortie de liquide de lavage (138), où, de préférence, une pièce de fixation pour une liaison amovible d'un système de liaison en forme de tube et laissant passer le liquide est disposée dans l'orifice d'entrée de liquide de lavage (16, 66, 116) pour l'évacuation du liquide de lavage hors du corps de prothèse, où l'orifice de sortie de liquide de lavage (138) ne peut pas être fermé par le système de liaison raccordé.

16. Entretoise d'articulation de la hanche selon l'une des revendications précédentes, **caractérisée en ce que**
l'espace creux (28, 78) ouvert des deux côtés du manchon d'ancrage (7, 57, 107) n'est pas relié à l'intérieur du corps de prothèse en laissant passer le liquide avec l'orifice d'entrée de liquide de lavage (136) et de préférence n'est également pas relié avec l'orifice de sortie de liquide de lavage (138).
